(19) Europäisches Patentamt
European Patent Office
Office européen des brevets

(11) Veröffentlichungsnummer: **0 361 161 B1**

(12) # EUROPÄISCHE PATENTSCHRIFT

(45) Veröffentlichungstag der Patentschrift: **30.03.94**

(51) Int. Cl.[5]: **C07D 231/54**, A01N 43/56, C07D 403/04, C07D 401/04, C07D 491/04

(21) Anmeldenummer: **89116515.1**

(22) Anmeldetag: **07.09.89**

Die Akte enthält technische Angaben, die nach dem Eingang der Anmeldung eingereicht wurden und die nicht in dieser Patentschrift enthalten sind.

(54) **Naphthindazol-4,9-chinone und ihre Verwendung zur Bekämpfung unerwünschten Pflanzenwuchses.**

(30) Priorität: **15.09.88 DE 3831332**

(43) Veröffentlichungstag der Anmeldung:
**04.04.90 Patentblatt 90/14**

(45) Bekanntmachung des Hinweises auf die Patenterteilung:
**30.03.94 Patentblatt 94/13**

(84) Benannte Vertragsstaaten:
**CH DE ES FR GB IT LI NL**

(56) Entgegenhaltungen:
**CH-A- 496 056**
**DE-A- 1 595 986**
**DE-A- 2 107 053**

**JUSTUS LIEBIGS ANNALEN DER CHEMIE, Band 1985, Nr. 2, 1985, Seiten 251-274, Weinheim, DE; H. LAATSCH: "Dimere Naphthochinone, XII. über die Selektivität der Addition von Diazomethan an benzoid substituierte 1,4-Naphthochinone"**

**CHEMISCHE BERICHTE, Band 97, Nr. 9, Seiten 2555-2566, Weinheim, DE; H. BROCKMANN et al.: "Diazomethan-Abbau von Bis-alpha-naphthochinon"**

**CHEM. BERICHTE; 101, Seiten 1987-1990, (1968)**

(73) Patentinhaber: **BASF Aktiengesellschaft**
**Carl-Bosch-Strasse 38**
**D-67063 Ludwigshafen(DE)**

(72) Erfinder: **Ditrich, Klaus, Dr.**
**Paray-Le-Monial-Strasse 12**
**D-6702 Bad Duerkheim(DE)**
Erfinder: **Hamprecht, Gerhard, Dr.**
**Rote-Turm-Strasse 28**
**D-6940 Weinheim(DE)**
Erfinder: **Wuerzer, Bruno, Dr.**
**Ruedigerstrasse 13**
**D-6701 Otterstadt(DE)**
Erfinder: **Meyer, Norbert, Dr.**
**Dossenheimer Weg 22**
**D-6802 Ladenburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft Benzindazol-4,9-chinone, Verfahren zu ihrer Herstellung und ihre Verwendung zur Bekämpfung unerwünschten Pflanzenwuchses.

Es ist bereits bekannt geworden, daß Indazolchinon-Derivate herbizide Eigenschaften besitzen (DE-A-2 107 053). Weiterhin sind Benzindazol-4,9-chinone aus Chem. Ber. 97, 2555, (1964) sowie aus Liebigs Ann. Chem. 1985, 251 bekannt, über ihre Verwendung ist jedoch nichts ausgesagt. Gemäß Chem. Ber. 101, 1987-1990 (1980) eignet sich 1-Vinyl-benzindazol-4,9-chinonfür die radikalische Polymerisation.

Es wurde gefunden, daß Benzindazol-4,9-chinone der Formel

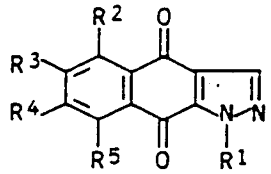

(Ia),

in der

| | |
|---|---|
| $R^1$ | Wasserstoff, $C_1$-$C_{10}$-Alkyl, $C_2$-$C_{10}$-Alkenyl, $C_2$-$C_{10}$-Alkinyl, $C_1$-$C_{10}$-Halogenalkyl, $C_1$-$C_{10}$-Hydroxyalkyl, $C_2$-$C_{14}$-Alkoxyalkyl, $C_2$-$C_{14}$-Alkylthioalkyl, $C_3$-$C_7$-Cycloalkyl, $C_1$-$C_4$-Alkylcarbonyloxy-$C_1$-$C_4$-alkyl, $C_1$-$C_4$-Alkylaminocarbonyloxy-$C_1$-$C_4$-alkyl, gegebenenfalls durch Halogen, $C_1$-$C_4$-Alkyl oder $C_1$-$C_4$-Alkoxy im Phenylrest substituiertes Phenylaminocarbonyloxy-$C_1$-$C_4$-alkyl, gegebenenfalls durch Halogen, $C_1$-$C_4$-Alkyl oder $C_1$-$C_4$-Alkoxy im Phenylrest substituiertes Phenylsulfonyloxy-$C_1$-$C_4$-Alkyl, $C_1$-$C_4$-Alkoxycarbonyl-$C_1$-$C_4$-alkyl, Hydroxycarbonyl$C_1$-$C_4$-alkyl, Aminocarbonyl-$C_1$-$C_4$-alkyl, $C_1$-$C_4$-Alkylaminocarbonyl-$C_1$-$C_4$-alkyl, Di-($C_1$-$C_4$-Alkyl)-aminocarbonyl-$C_1$-$C_4$-alkyl, Halogen-$C_3$-$C_4$-alkinyl, einen gegebenenfalls durch Halogen oder $C_1$-$C_4$-Alkyl substituierten Heteroarylrest mit einem oder zwei Stickstoffatomen, wie Pyrimidyl, Pyridyl, Imidazolyl, einen gegebenenfalls durch Halogen, Hydroxy, Nitro, Amino, $C_1$-$C_4$-Alkylamino, Di-$C_1$-$C_4$-alkylamino, Cyano, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Alkylthio oder $C_1$-$C_4$-Halogenalkylthio substituierten Phenylrest oder einen gegebenenfalls durch halogen substituierten Benzylrest und |
| $R^2$, $R^3$, $R^4$ und $R^5$ | unabhängig voneinander Wasserstoff, Halogen, Nitro, Cyano, Hydroxy, Amino, $C_1$-$C_4$-Alkylamino, Di-$C_1$-$C_4$-alkylamino, $C_1$-$C_6$-Alkyl, $C_1$-$C_5$-Halogenalkyl, $C_1$-$C_5$-Alkoxy, $C_1$-$C_5$-Halogenalkoxy, $C_1$-$C_5$-Alkylthio, $C_1$-$C_5$-Halogenalkylthio, $C_2$-$C_{10}$-Alkoxyalkyl, Carboxyl, $C_2$-$C_6$-Alkoxycarbonyl, $C_2$-$C_6$-Alkanoyloxy, $C_2$-$C_6$-Halogenalkanoyloxy, $C_1$-$C_4$-Alkylcarbonylamino, $C_1$-$C_4$-Alkylaminocarbonyl, Di-($C_1$-$C_4$-Alkyl)-aminocarbonyl oder einen gegebenenfalls durch Halogen, Trifluormethyl, Nitro, Cyano, Amino, $C_1$-$C_4$-Alkoxy, $C_1$-$C_4$-Alkylthio oder $C_1$-$C_5$-Halogenalkylthio substituierten Phenyl- oder Heteroarylrest, wie Pyridyl, Thienyl, Furyl, Benzimidazol-2-yl oder Pyrimid-2-yl, bedeuten |

und außerdem

$R^3$ und $R^4$ gemeinsam mit den beiden Kohlenstoffatomen des Phenylringes, an die sie gebunden sind, einen gegebenenfalls durch Halogen, Nitro, Cyano, Amino, Hydroxy, Trifluormethyl, $C_1$-$C_5$-Alkyl, $C_1$-$C_5$-Alkoxy, $C_1$-$C_5$-Halogenalkylthio, $C_1$-$C_5$-Halogenalkoxy oder $C_1$-$C_5$-Alkylthio substituierten Benzol- oder Naphthalinring bilden, oder einen heterocyclischen Ring, ausgewählt aus der Gruppe 1,4-Dioxan, 1,3-Dioxolan, Pyrazol, Indol, Thiophen, Triazol und Piperazin, 2,2-Dimethyl-1,3-dioxolan, 1-Methylpyrazol, 1-Methyl-indol, N,N'-Dimethylpiperazin, 2,2-Diphenyl-1,3-dioxolan, 2-Oxo-1,3-dioxolan bilden,

gute herbizide Eigenschaften besitzen und gegenüber Kulturpflanzen selektiv wirksam sind.

In Formel Ia haben die Reste $R^1$, $R^2$, $R^3$, $R^4$ und $R^5$ die folgenden Bedeutungen:

| | |
|---|---|
| $R^1$: | Wasserstoff, $C_1$-$C_{10}$-Alkyl, vorzugsweise $C_1$-$C_4$-Alkyl, $C_2$-$C_{10}$-Alkenyl, vorzugsweise $C_2$-$C_4$-Alkenyl, $C_2$-$C_{10}$-Alkinyl, vorzugsweise $C_2$-$C_4$-Alkinyl, $C_1$-$C_{10}$-Hydroxyalkyl, vorzugsweise $C_1$-$C_4$-Hydroxyalkyl, $C_2$-$C_{14}$-Alkoxyalkyl, vorzugsweise $C_2$-$C_6$-Alkoxyalkyl, $C_2$-$C_{14}$-Alkylthioalkyl, vorzugsweise $C_2$-$C_6$-Alkylthioalkyl, $C_3$-$C_7$-Cycloalkyl, vorzugsweise $C_3$-$C_6$-Cycloalkyl, $C_1$-$C_4$-Alkylcarbonyloxy-$C_1$-$C_4$-alkyl, $C_1$-$C_4$-Alkylaminocarbonyloxy-$C_1$-$C_4$-alkyl, gegebenenfalls durch Halogen, $C_1$-$C_4$-Alkyl oder $C_1$-$C_4$-Alkoxy im |

Phenylrest substituiertes Phenylaminocarbonyloxy-$C_1$-$C_4$-alkyl, gegebenenfalls durch Halogen, $C_1$-$C_4$-Alkyl oder $C_1$-$C_4$-Alkoxy im Phenylrest substituiertes Phenylsulfonyloxy-$C_1$-$C_4$-Alkyl, $C_1$-$C_4$-Alkoxycarbonyl-$C_1$-$C_4$-alkyl, Hydroxycarbonyl-$C_1$-$C_4$-alkyl, Aminocarbonyl-$C_1$-$C_4$-alkyl, $C_1$-$C_4$-Alkylaminocarbonyl-$C_1$-$C_4$-alkyl, Di-($C_1$-$C_4$-Alkyl)-aminocarbonyl-$C_1$-$C_4$-alkyl, Halogen-$C_3$-$C_4$-alkinyl, einen gegebenenfalls durch Halogen oder $C_1$-$C_4$-Alkyl substituierten Heteroarylrest mit einem oder zwei Stickstoffatomen, z.B. Pyrimidyl, Pyridyl, Imidazolyl, einen gegebenenfalls einfach oder mehrfach durch Halogen, Hydroxy, Nitro, Amino, $C_1$-$C_4$-Alkylamino, Di-$C_1$-$C_4$-alkylamino, Cyano, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio substituierten Phenylrest, einen gegebenenfalls durch Halogen im Phenylteil einfach oder mehrfach substituierten Benzylrest, beispielsweise Methyl, Ethyl, n-Propyl, Isopropyl, n-Butyl, Isobutyl, sec.-Butyl, tert.-Butyl, n-Pentyl, tert.-Amyl, n-Hexyl, Pentyl-3, 1,2-Dimethyl-n-propyl, 1,3-Dimethyl-n-butyl, 1-Ethyl-2-methyl-n-propyl, 1,2,2-Trimethyl-n-propyl, 1,2-Dimethyl-4-hexyl, Allyl, Methallyl, Crotyl, 2-Ethyl-hex-2-enyl, Hex-5-enyl, 2-Methyl-but-2-enyl, 2-Methyl-but-3-enyl, But-1-en-3-yl, 2-Methyl-but-1-en-4-yl, 2-Methyl-but-2-en-4-yl, 3-Methyl-but-1-en-3-yl, Propargyl, But-1-in-3-yl, But-2-inyl, 2-Chlorethyl, 2-Chlor-n-propyl, 3-Chlor-n-propyl, 2-Chlor-sec.-butyl, 2-Chlor-iso-butyl, 2-Fluor-sec.-butyl, 2-Fluor-isobutyl, 2-Fluor-isopropyl, Chlor-tert.-butyl, 2,2,2-Trifluorethyl, Hydroxymethyl, 2-Hydroxyethyl, 3-Hydroxy-n-propyl, 2-Hydroxy-iso-propyl, 2-Hydroxy-n-butyl, 3-Hydroxy-n-butyl, 4-Hydroxy-n-butyl, 2-Hydroxy-iso-butyl, 2-Methoxy-ethyl, 2-Ethoxyethyl, 3-Methoxy-n-propyl, 2-Methoxy-isopropyl, 3-Methoxy-n-butyl, 1-Methoxy.sec.-butyl, Methoxy-tert.-butyl, Ethoxy-tert.-butyl, 2-Methoxy-n-butyl, 4-Methoxy-n-butyl, Cyclopropyl, Cyclopentyl, Cyclohexyl, 2-Methylmercapto-ethyl, 2-Ethylmercaptoethyl, 3-Methylmercapto-n-propyl, 3-Methylmercapto-n-butyl, 1-Methylmercapto-sec.-butyl, Methylmercapto-tert.-butyl, 2-Methylmercapto-n-butyl, Phenyl, 4-Chlorphenyl, 3,4-Dichlorphenyl, o-, m-, p-tert.-Butylphenyl, o-, m-, p-Methoxyphenyl, o-, m-, p-Methylphenyl, 4-Methoxy-3-chlorphenyl, 2-Methyl-4-chlorphenyl, Benzyl, 2,6-Dichlorbenzyl, 2-Chlor-6-fluorbenzyl, 2,6-Difluorbenzyl, o-, m-, p-Chlorbenzyl.

$R^2$, $R^3$, $R^4$, $R^5$: Wasserstoff, Halogen, wie Fluor, Chlor, Brom, Jod, Nitro, Cyano, Hydroxy, Amino, $C_1$-$C_4$-Alkylamino oder Di-$C_1$-$C_4$-alkylamino, $C_1$-$C_6$-Alkyl, vorzugsweise $C_1$-$C_4$-Alkyl, $C_1$-$C_5$-Halogenalkyl, vorzugsweise $C_1$-$C_3$-Halogenalkyl, $C_1$-$C_5$-Alkoxy, vorzugsweise $C_1$-$C_3$-Alkoxy, $C_1$-$C_5$-Halogenalkoxy, vorzugsweise $C_1$-$C_3$-Halogenalkoxy, $C_1$-$C_5$-Alkylthio, vorzugsweise $C_1$-$C_3$-Alkylthio, $C_1$-$C_5$-Halogenalkylthio, vorzugsweise $C_1$-$C_3$-Halogenalkylthio, $C_2$-$C_{10}$-Alkoxyalkyl, vorzugsweise $C_2$-$C_6$-Alkoxyalkyl, Carboxyl, $C_2$-$C_6$-Alkoxycarbonyl, vorzugsweise $C_2$-$C_4$-Alkoxycarbonyl, $C_2$-$C_6$-Alkanoyloxy, vorzugsweise $C_2$-$C_4$-Alkanoyloxy, $C_3$-$C_6$-Halogenalkanoyloxy, vorzugsweise $C_2$-$C_4$-Halogenalkanoyloxy, $C_1$-$C_4$-Alkylcarbonylamino, $C_1$-$C_4$-Alkylaminocarbonyl, Di-($C_1$-$C_4$-Alkyl)aminocarbonyl, einen gegebenenfalls durch Halogen, vorzugsweise Chlor oder Fluor, Trifluormethyl, Nitro, Cyano, Amino, $C_1$-$C_4$-Alkoxy, $C_1$-$C_4$-Alkylthio oder $C_1$-$C_5$-Halogenalkylthio substituierten Phenyl- oder Heteroarylrest, wie Pyridyl, Pyrimidyl, Thienyl, Furyl oder Benzimidazolyl, z.B. Methylamino, Dimethylamino, Ethylamino, Diethylamino, Isopropylamino, Diisopropylamino, Methylethylamino, n-, iso-, tert.-Butylamino, Methyl, Ethyl, n-Propyl, Isopropyl, n-Butyl, Isobutyl, sec.-Butyl, tert.-Butyl, n-Pentyl, tert.-Amyl, n-Hexyl, Pentyl-3, 1,2-Dimethyl-n-propyl, 1,3-Dimethyl-n-butyl, 1-Ethyl-2-methyl-n-propyl, 1,2,2-Trimethyl-n-propyl, 1,2-Dimethyl-n-hexyl, Allyl, Methallyl, Crotyl, 2-Ethyl-hex-2-enyl, Hex-5-enyl, 2-Methyl-but-2-enyl, 2-Methyl-but-3-enyl, But-1-en-3-yl, 2-Methyl-but-1-en-4-yl, 2-Methyl-but-2-en-4-yl, 3-Methyl-but-1-en-3-yl, Propargyl, But-1-in-3-yl, But-2-inyl, Fluormethyl, Chlormethyl, Trifluormethyl, Difluormethyl, Trichlormethyl, Dichlormethyl, 2-Chlor-ethyl, 2-Chlor-n-propyl, 3-Chlor-n-propyl, 2-Chlor-isopropyl, Pentafluorethyl, 2,2,2-Trifluorethyl, 2-Fluor-isopropyl, 3-Fluor-n-propyl, 2-Fluorethyl, Methoxy, Ethoxy, n-, iso-Propoxy, n-, iso-, tert.-Butoxy, Pentoxy, Difluormethoxy, Trifluormethoxy, Trichlormethoxy, Methylthio, Ethylthio, n-, iso-Propyl-thio, n-, iso-, tert.-Butylthio, Difluormethylthio, Trifluormethylthio, Methoxymethyl, Ethoxymethyl, 2-Methoxy-ethyl, 3-Methoxy-n-propyl, 2-Methoxy-isopropyl, 2-Ethoxy-ethyl, 3-Ethoxy-n-propyl, 2-Ethoxy-isopropyl, Methoxycarbonyl, Ethoxy-carbonyl, n-, iso-Propoxycarbonyl, n-, iso-, tert.-Butoxycarbonyl, 2-Methoxy-ethoxycarbonyl, Ethoxy-methoxycarbonyl, 2-Ethoxy-ethoxycarbonyl, Phenyl, o-, m-, p-Fluorphenyl, o-, m-, p-Chlorphenyl,

2,4-Difluorphenyl, 2,4-p-Trifluormethylphenyl, 2-, 3- und 4-Nitrophenyl, 2- 3- und 4-Cyanophenyl, 2-, 3- und 4-Aminophenyl, 2-, 3- und 4-Methoxyphenyl, 2, 4-Dimethoxyphenyl, 2,4,5-Trimethoxyphenyl, 2-, 3- und 4-Thiomethylphenyl, Heteroaryl wie Pyrid-2-yl, Pyrid-3-yl, Pyrid-4-yl, Thien-2-yl, Thien-3-yl, Fur-2-yl, Fur-3-yl, Benzimidazol-2-yl, 3-Chlor-pyrid-6-yl, 2-Methyl-fur-5-yl, 2-Methyl-thien-5-yl und 4,6-Dimethyl-pyrimid-2-yl, Acetyl, Propionyl, Butyryl, Isobutyryl, Valeryl, Isovaleryl, Pivalyl, Chloracetyl, Dichloracetyl, Trichloracetyl, Fluoracetyl, Difluoracetyl, Trifluoracetyl, 3,3,3-Trifluorpropionyl, Pentafluorpropionyl, 2-Chlor-propionyl, 2,2-Dichlor-propionyl, 2-Fluor-propionyl, 2,2-Difluor-propionyl.

$R^3$ und $R^4$ können außerdem gemeinsam mit den beiden Kohlenstoffatomen des Phenylrings, an die sie gebunden sind, einen gegebenenfalls durch Fluor, Chlor, Brom, Nitro, Cyano, Amino, Hydroxy, Trifluormethyl, $C_1$-$C_5$-Alkyl, vorzugsweise $C_1$-$C_3$-Alkyl, $C_1$-$C_5$-Alkoxy, $C_1$-$C_5$-Halogenalkylthio, $C_1$-$C_5$-Halogenalkoxy oder $C_1$-$C_5$-Alkylthio, vorzugsweise $C_1$-$C_3$-Alkylthio, substituierten Benzol- oder Naphthalinring bilden.

Heterocyclische Ringe, die $R^3$ und $R^4$ gemeinsam mit den beiden C-Atomen des Phenylringes, an die sie gebunden sind, bilden, sind 1,4-Dioxan, 1,3-Dioxolan, Pyrazol, Indol, Thiophen, Triazol und Piperazin. Beispiele für die entsprechenden substituierten Ringe sind 2,2-Dimethyl-1,3-dioxolan, 1-Methylpyrazol, 1-Methyl-indol, N,N'-Dimethylpiperazin, 2,2-Diphenyl-1,3-dioxolan, 2-Oxo-1,3-dioxolan.

Benzindazol-4,9-chinone der Formel Ia, in der $R^1$ $C_1$-$C_4$-Alkyl oder $C_2$-$C_4$-Alkenyl bedeutet, sind bevorzugt. Weiterhin sind solche Verbindungen der Formel Ia bevorzugt, in der die Substituenten $R^2$ bis $R^5$ unabhängig voneinander Wasserstoff, Hydroxy, Halogen, Nitro, Cyano, Amino, $C_1$-$C_4$-Alkylamino oder Di-$C_1$-$C_4$-Alkylamino bedeuten. Insbesondere bevorzugt sind Verbindungen mit den Resten Wasserstoff, Hydroxy oder Halogen für $R^2$ bis $R^5$.

Die Benzindazol-4,9-chinone der Formel

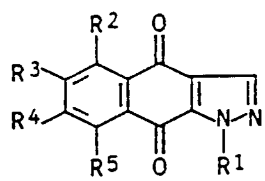

(I),

in der

$R^1$, $R^2$, $R^3$, $R^4$ und $R^5$ die für Formel Ia angegebenen Bedeutungen haben, mit der Maßgabe, daß $R^1$ nicht Wasserstoff oder Methyl bedeutet und daß 1-Vinylbenzindazol-4,9-chinon ausgenommen ist, sind neu.

Man erhält die Benzindazol-4,9-chinone der Formel I, indem man die 5-Arylmethylpyrazol-4-carbonsäurehalogenide der Formel

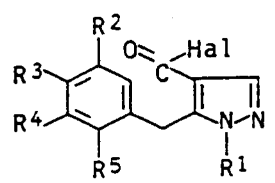

(II),

in der $R^1$, $R^2$, $R^3$, $R^4$ und $R^5$ die oben angegebenen Bedeutungen haben und Hal für Halogen steht, in einem inerten Lösungsvermittler in Gegenwart eines Friedel-Crafts-Katalysators zur Reaktion bringt, und die so erhaltenen Verbindungen der Formel

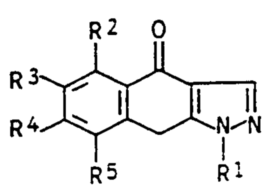

(III),

in der $R^1$, $R^2$, $R^3$, $R^4$ und $R^5$ die oben angegebenen Bedeutungen haben, in Gegenwart eines Verdünnungsmittels mit Oxidationsmitteln in die Benzindazol-4,9-chinone der Formel I überführt.

Geeignete inerte Lösungsvermittler hierfür sind Schwefelkohlenstoff, Nitromethan, Nitrobenzol, 1,2-Dichlorethan, 1,1,2,2-Tetrachlorethan, Sulfolan, Trichlorethylen und 1,1,1-Trichlorethan.

Als Friedel-Crafts-Katalysatoren kommen Aluminiumtrichlorid, Bortrifluorid, Zinntetrachlorid, Titantetrachlorid, Zinkchlorid, Bortrichlorid, Trifluoressigsäure, Schwefelsäure, Aluminiumbromid, Galliumchlorid, Eisen(III)chlorid, Antimonpentachlorid, Antimontrichlorid, Zirkontetrachlorid in Betracht. Die Katalysatormenge beträgt 5 bis 500, vorzugsweise 100 bis 250 Mol%, bezogen auf eingesetztes Säurechlorid der Formel II.

Die Umsetzung verläuft vorteilhaft bei einer Temperatur zwischen 0 und 150 °C.

Die Oxidation der Verbindungen III wird zweckmäßigerweise in einem inerten Verdünnungsmittel, wie Essigsäure oder alkoholischer Alkalilauge, gegebenenfalls im Gemisch mit Wasser bei einer Temperatur zwischen 0 und 120 °C durchgeführt. Geeignete Oxidationsmittel sind Wasserstoffperoxid oder Chrom(IV)-oxid.

Die Oxidation wird zweckmäßigerweise so durchgeführt, daß man die Ausgangsverbindungen III in verdünnten, wässrig alkoholischen Lösungen von Alkalihydroxiden bei Temperaturen zwischen 0 °C und 120 °C, vorzugsweise 50 °C und 100 °C, löst und die so erhaltenen Mischungen mit überschüssiger wässriger Wasserstoffperoxidlösung versetzt. Die Umsetzung ist im allgemeinen nach 4 bis 8, höchstens 24 Stunden beendet. Das feste Endprodukt wird abgesaugt und kann zur Reinigung gegebenenfalls umkristallisiert oder chromatographiert werden.

Die Synthese der 5-Arylmethyl-pyrazol-4-yl-carbonsäurehalogenide II kann nach an sich bekannter weise nach folgendem Schema erfolgen:

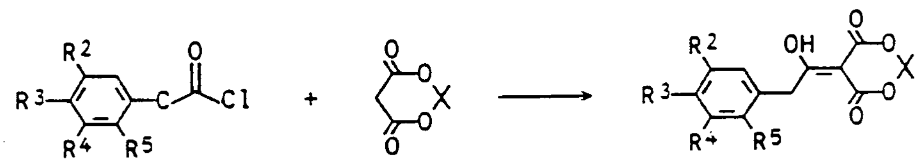

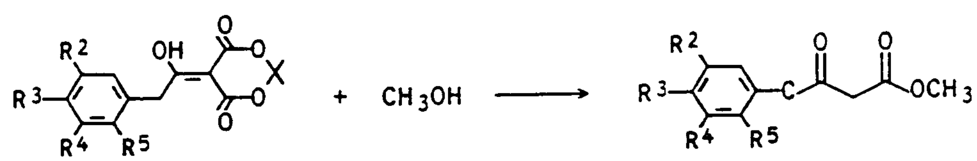

(Y. Oikana, K. Sugano und O. Yonemitsu, J. Org. Chem. 43, 2087 (1978)

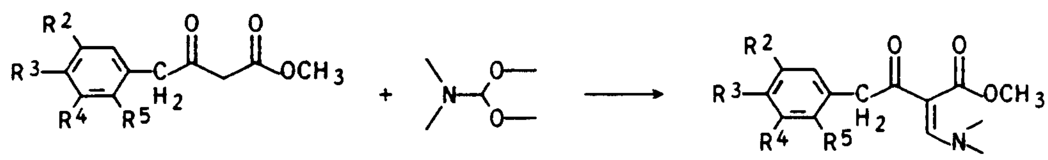

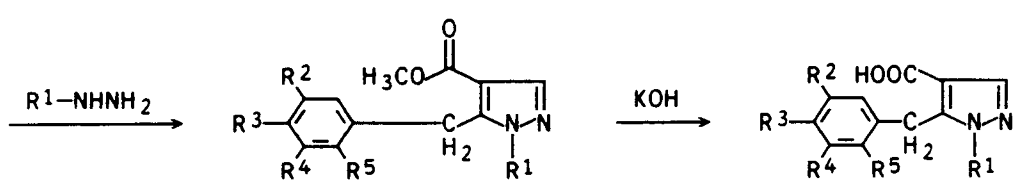

(G.Menozzi, L.Mosti und P.Schenone, J.Heterocyclic Chem. 24, 1969 (1987))

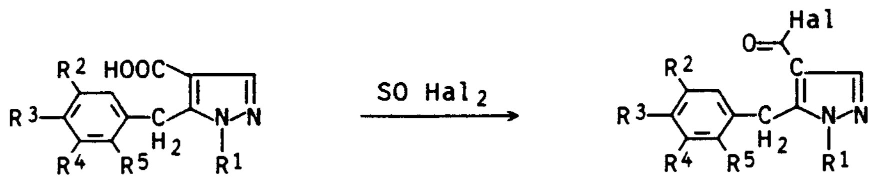


(II)

Man erhält die Benzindazol-4,9-chinone der Formel I ebenfalls durch Umsetzung von substituierten 1,4-Naphthochinonen der Formel

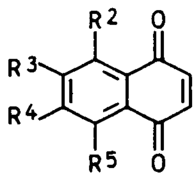

(IV),

in der $R^2$, $R^3$, $R^4$ und $R^5$ die oben angegebenen Bedeutungen haben,
mit Diazomethan in Gegenwart von inerten Verdünnungsmitteln, wie zum Beispiel aliphatischen oder alicyclischen Ethern, Oxidation der dabei anfallenden Verbindungen der Formel

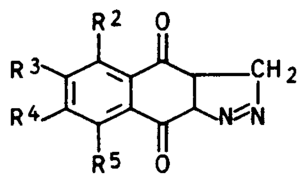

(V),

in der $R^2$, $R^3$, $R^4$ und $R^5$ die oben angegebenen Bedeutungen haben,
in einem inerten Verdünnungsmittel und Alkylierung der dabei anfallenden Benzindazol-4,9-chinone der Formel

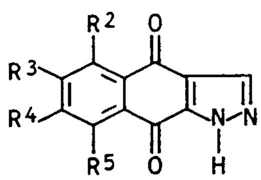

(VI),

in der $R^2$, $R^3$, $R^4$ und $R^5$ die oben angegebenen Bedeutungen haben,
mit einem Alkylierungsmittel der Formel

$R^1$-X (VII),

in der
$R^1$ die oben angegebene Bedeutung hat, und
X für p-Toluolsulfonat, Mesylat, Brosylat, Halogen oder für eine Gruppe der Formel

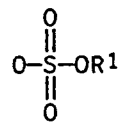 ,

in der $R^1$ die oben angegebene Bedeutung hat, steht,
in einem Verdünnungsmittel in Gegenwart einer Base bei einer Temperatur zwischen -30 °C und 100 °C.

Die Umsetzung der 1,4-Naphthochinone IV mit Diazomethan läßt sich in an sich üblicher Weise durchführen (Houben-Weyl, Methoden der org. Chemie, Bd. 7/3a, S. 553 (1977)). Die Oxidation der Verbindungen V gelingt u.a. mit Wasserstoffperoxid, Chrom(VI)-oxid oder Luftsauerstoff in einem inerten Verdünnungsmittel, wie verdünnter Essigsäure oder Methyl-t-butylether.

Die Alkylierung der Benzindazol-4,9-chinone VI mit dem Alkylierungsmittel VII verläuft in an sich bekannter Weise in einem Verdünnungsmittel, wie beispielsweise Wasser oder Aceton oder Mischungen daraus, in Gegenwart einer Base, wie Alkali- oder Erdalkalihydroxiden, Alkali- oder Erdalkalicarbonaten bzw. Alkali- oder Erdalkalihydrogencarbonaten. Vorzugsweise arbeitet man bei einer Temperatur zwischen 10 und 30 °C.

Alle übrigen Ausgangsverbindungen bzw. Reaktionsprodukte, deren Herstellung nicht im einzelnen beschrieben ist, sind entweder bekannt oder lassen sich nach im Prinzip bekannten Methoden herstellen.

Herstellungsbeispiele

Beispiel 1

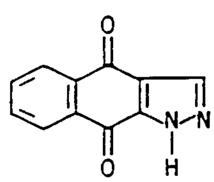

Eine Suspension von 20,6 g (0,13 mol) 1,4-Naphthochinon in 500 ml Methyl-t-butylether wird unter Schutzgasatmosphäre bei 0 °C mit 400 ml einer $3{,}5 \times 10^{-4}$ molaren Lösung (0,14 mol) von Diazomethan in Diethylether versetzt. Nach beendeter Zugabe läßt man die Suspension auf Raumtemperatur erwärmen und leitet über Nacht Luft durch das Reaktionsgemisch. Dann wird der ausgefallene Niederschlag abgesaugt, mit 100 ml Methyl-t-butylether gewaschen und der Rückstand in 75 ml heißem Ethanol verrührt. Man saugt ab, wäscht mit 100 ml Ethanol und trocknet.
Ausbeute: 12,1 g (47 % der Th.)
Schmelzpunkt: 180 °C Zersetzung

Beispiel 2

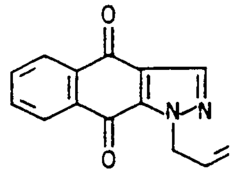

6,0 g (0,03 mol) Benzindazol-4,9-chinon (Beispiel 1) werden in 100 ml 80 %igem, wässrigen Aceton suspendiert und bei Raumtemperatur nacheinander mit 3,2 g (0,03 mol) Natriumcarbonat und 7,3 g (0,06 mol) 1-Brom-2-propen versetzt. Man rührt über Nacht nach und erhitzt dann drei Stunden unter Rückfluß. Man läßt abkühlen, gibt erneut 1,1 g (0,01 mol) Natriumcarbonat und 2,4 g (0,02 mol) 1-Brom-2-propen zu und erhitzt nochmals zwei Stunden unter Rückfluß. Danach wird unter vermindertem Druck eingeengt und viermal mit je 100 ml Dichlormethan extrahiert. Die vereinten Extrakte werden über Natriumsulfat getrocknet und eingeengt. Den kristallinen Rückstand chromatographiert man an Kieselgel (Elutionsmittel: Cyclohexan/Essigester) und erhält so 4,6 g (64 % der Theorie) 1-(Propen-2-yl)-benzindazol-4,9-chinon vom Schmelzpunkt 105-108°C.

Beispiel 3

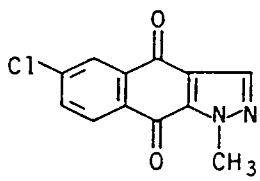

a) 1,25 g (0,005 mol) 5-(4-Chlorphenyl-methyl)-1-methyl-pyrazol-4-yl-carbonsäure (hergestellt analog: G. Menozzi, L. Mosti und P. Schenone, J. Heterocyclic Chem. 24, 1669 (1987)) werden in 2 ml Thionylchlorid gelöst und eine Stunde unter Rückfluß erhitzt. Danach wird überschüssiges Thionylchlorid unter vermindertem Druck (12 Torr) entfernt, der Rückstand mit 20 ml Diethylether aufgenommen und mit 2,0 g Aktivkohle gerührt. Nach 30 Minuten wird filtriert und das Filtrat im Vakuum konzentriert. Man behält 1,25 g (93 % der Theorie) Säurechlorid als farbloses Öl zurück.

b) 1,25 g (4,6 mmol) 5-(4-Chlorphenyl-methyl)-1-methyl-pyrazol-4-yl-carbonsäurechlorid werden in 5 ml Nitromethan gelöst und zu einer auf 0°C gekühlten Lösung von 1,85 g (14 mmol) Aluminiumchlorid in 20 ml Nitromethan gegeben. Man rührt über Nacht bei Raumtemperatur nach und gießt das Gemisch dann in eine Mischung aus 50 g Eis und 20 ml konzentrierter Salzsäure. Die organische Phase wird abgetrennt, die wässrige Phase noch zweimal mit je 50 ml Dichlormethan extrahiert und die vereinten Extrakte über Natriumsulfat getrocknet. Nach dem Entfernen des Lösungsmittels unter vermindertem Druck bleiben 1,10 g (100 % der Theorie) 6-Chlor-9-hydro-1-methyl-benzindazol-4-on vom Schmelzpunkt > 270°C zurück.

$^1$H-NMR (250 MHz, DMSO-$d_6$): 4.05 (s; 3H), 4.45 (s; 2H), 7.35 (dd, J = 9.2 und 0.2 Hz; 1H), 7.90 (d,J = 9.0 Hz; 1H), 8.20 (d, J = 0.2 Hz; 1H), 8.45 (s;1H)

c) 1,10 g (4,6 mmol) 6-Chlor-9-hydro-1-methyl-benzindazol-4-on werden in einer Mischung aus 10 ml 10 %iger Natronlauge und 50 ml Methanol in der Wärme gelöst (80°C). Man läßt auf 60°C abkühlen und gibt dann 5 ml einer 30 %igen Lösung von Wasserstoffperoxid zu. Anschließend rührt man über Nacht bei Raumtemperatur nach und saugt dann das ausgefallende Chinon ab. Man erhält so 0,85 g (57 % der Theorie) 1-Methyl-6-chlor-benzindazol-4,9-chinon vom Schmelzpunkt: 219°C.

$^1$H-NMR (250 MHz, $CDCl_3$): 4.35 (s;3H), 7.72 (d,J = 9.0 Hz; 1H), 8.05 (s; 1H), 8.18 (s;1H); 8.18 (d, J = 9.0 Hz; 1H).

Die folgenden Verbindungen der Formel I bzw. Ia können in analoger Weise erhalten werden:

| Beispiel Nr. | $R^1$ | $R^2$-$R^5$ | Schmelzpunkt [°C] |
|---|---|---|---|
| 4 | $CH_3$ | H | 181 |
| 5 | $CH_2CH_3$ | H | 151-152 |
| 6 | i-Propyl | H | 126 |
| 7 | n-Propyl | H | |
| 8 | n-Butyl | H | 69 |
| 9 | t-Butyl | H | 152 |
| 10 | 2-Pentyl | H | 164 |
| 11 | cyclo-Pentyl | H | |
| 12 | cyclo-Hexyl | H | 148 |
| 13 | $CH_2$-$CF_3$ | H | 171 |
| 14 | $CH_2$-OH | H | |
| 15 | $CH_2$-$CH_2$-OH | H | 158 |
| 16 | 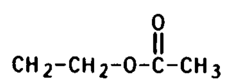 | H | 109 |
| 17 | 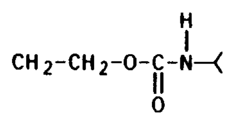 | H | 116 |
| 18 | 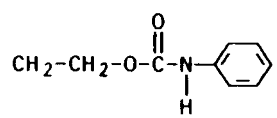 | H | 179 |
| 19 | 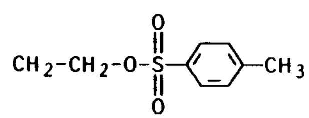 | H | 155 |

| Beispiel Nr. | $R^1$ | $R^2$-$R^5$ | Schmelzpunkt [°C] |
|---|---|---|---|
| 20 | $CH_2-COOC_2H_5$ | H | 149 |
| 21 | $CH_2-COOH$ | H | |
| 22 | $CH_2C(=O)-NH2$ | H | |
| 23 | 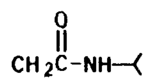 | H | |
| 24 | $CH_2-C(=O)-N(CH_3)_2$ | H | |
| 25 | $CH_2CH_2-S-CH_3$ | H | |
| 26 | $CH_2-S-CH_3$ | H | |
| 27 | $CH_2-C\equiv CH$ | H | 193 |
| 28 | $CH_2-C\equiv C-Br$ | H | |
| 29 | Phenyl | H | 221 |
| 30 | Benzyl | H | 154 |
| 31 | 2-(4,6-Dimethyl-pyrimidinyl) | H | 237 |
| 32 | 2-Pyridinyl | H | 221 |
| 33 | 2-(2-Imidazolin) | H | |
| 34 | 2,4-Dichlorphenyl | H | |
| 35 | 2,4,6-Trichlorphenyl | H | |
| 36 | 4-Fluorphenyl | H | |
| 37 | 2,4-Difluorphenyl | H | |
| 38 | 2,4,6-Trifluorphenyl | H | |

| Beispiel Nr. | $R^1$ | $R^2$-$R^5$ | Schmelzpunkt [°C] |
|---|---|---|---|
| 39 | 4-Methoxyphenyl | H | |
| 40 | 2,4-Dimethoxyphenyl | H | |
| 41 | 4-Thiomethylphenyl | H | |
| 42 | 4-Trifluormethylphenyl | H | |

| Beispiel Nr. | $R^1$ | $R^2$ | $R^3$ | $R^4$ | $R^5$ | Schmelzpunkt [oC] |
|---|---|---|---|---|---|---|
| 43 | $CH_3$ | F | H | H | H | 195 |
| 44 | $CH_3$ | H | F | H | H | |
| 45 | $CH_3$ | H | H | F | H | 173 |
| 46 | $CH_3$ | H | H | H | F | |
| 47 | $CH_3$ | Cl | H | H | H | |
| 48 | $CH_3$ | H | Cl | H | H | 219 |
| 49 | $CH_3$ | H | H | Cl | H | 226 |
| 50 | $CH_3$ | H | H | H | Cl | |
| 51 | $CH_3$ | Br | H | H | H | |
| 52 | $CH_3$ | H | H | H | Br | 190-192 |
| 53 | $CH_3$ | Cl | H | H | Cl | |
| 54 | $CH_3$ | H | Cl | H | Cl | 230 |
| 55 | $CH_3$ | H | Cl | Cl | H | |
| 56 | $CH_3$ | F | H | H | F | |
| 57 | $CH_3$ | H | F | H | F | |
| 58 | $CH_3$ | H | F | F | H | |
| 59 | $CH_3$ | OH | H | H | H | 190 |
| 60 | $CH_3$ | H | OH | H | H | |
| 61 | $CH_3$ | H | H | OH | H | |
| 62 | $CH_3$ | H | H | H | OH | 212 |
| 63 | $CH_3$ | OH | H | H | OH | 224-227 |
| 64 | $CH_3$ | $OCH_3$ | H | H | H | |

| Beispiel Nr. | $R^1$ | $R^2$ | $R^3$ | $R^4$ | $R^5$ | Schmelzpunkt [oC] |
|---|---|---|---|---|---|---|
| 65 | $CH_3$ | H | $OCH_3$ | H | H | 193 |
| 66 | $CH_3$ | H | H | $OCH_3$ | H | 173 |
| 67 | $CH_3$ | H | H | H | $OCH_3$ | 230 |
| 68 | $CH_3$ | $OCH_3$ | H | $CH_3$ | H | 212-213 |
| 69 | $CH_3$ | OH | H | H | $OCH_3$ | |
| 70 | $CH_3$ | $OCH_3$ | H | H | OH | |
| 71 | $CH_3$ | $OCH_3$ | $OCH_3$ | H | $OCH_3$ | |
| 72 | $CH_3$ | OH | $OCH_3$ | H | $OCH_3$ | |
| 73 | $CH_3$ | $OCH_3$ | H | $OCH_3$ | H | 212 |
| 74 | $CH_3$ | $OCH_3$ | H | $OCH_3$ | OH | |
| 75 | $CH_3$ | OH | H | $OCH_3$ | H | 208 |
| 76 | $CH_3$ | H | $OCH_3$ | H | $OCH_3$ | |
| 77 | $CH_3$ | H | $OCH_3$ | H | OH | |
| 78 | $CH_3$ | H | $O-CH_2-CH_2O$ | | H | |
| 79 | $CH_3$ | H | $O-CH_2-O$ | | H | 252 |
| 80 | $CH_3$ | H | OH | OH | H | |
| 81 | $CH_3$ | $S-CH_3$ | H | H | H | |
| 82 | $CH_3$ | H | $S-CH_3$ | H | H | |
| 83 | $CH_3$ | H | H | $S-CH_3$ | H | |
| 84 | $CH_3$ | H | H | H | $SCH_3$ | |
| 85 | $CH_3$ | $O-C(=O)-CH_3$ | H | H | H | 220 |

| Beispiel Nr. | $R^1$ | $R^2$ | $R^3$ | $R^4$ | $R^5$ | Schmelzpunkt [oC] |
|---|---|---|---|---|---|---|
| 86 | $CH_3$ | H | H | H | $O-\overset{O}{\overset{\parallel}{C}}-CH_3$ | |
| 87 | $CH_3$ | $CH_3$ | H | H | H | |
| 88 | $CH_3$ | H | $CH_3$ | H | H | 177 |
| 89 | $CH_3$ | H | H | $CH_3$ | H | 136 |
| 90 | $CH_3$ | H | H | H | $CH_3$ | 218 |
| 91 | $CH_3$ | $CH_3$ | H | H | $CH_3$ | 143 |
| 92 | $CH_3$ | $CF_3$ | H | H | H | |
| 93 | $CH_3$ | H | $CF_3$ | H | H | |
| 94 | $CH_3$ | H | H | $CF_3$ | H | |
| 95 | $CH_3$ | H | H | H | $CF_3$ | |
| 96 | $CH_3$ | H | $CH_3$ | H | OH | 154 |
| 97 | $CH_3$ | OH | H | $CH_3$ | H | 175 |
| 98 | $CH_3$ | H | $C_4H_4$ | | OH | 288 |
| 99 | $CH_3$ | H | $C_4H_4$ | | H | |
| 100 | $CH_3$ | OH | $C_4H_4$ | | H | |
| 101 | $CH_3$ | H | $C_4H_4$ | | $O-\overset{O}{\overset{\parallel}{C}}-CH_3$ | 253 |
| 102 | $CH_3$ | $O-\overset{O}{\overset{\parallel}{C}}-CH_3$ | $C_4H_4$ | | H | 231 |
| 103 | $CH_3$ | F | $C_4H_4$ | | OH | |

| Beispiel Nr. | $R^1$ | $R^2$ | $R^3$ | $R^4$ | $R^5$ | Schmelzpunkt [oC] |
|---|---|---|---|---|---|---|
| 104 | $CH_3$ | $NO_2$ | H | H | H | |
| 105 | $CH_3$ | H | H | H | $NO_2$ | |
| 106 | $CH_3$ | $NH_2$ | H | H | H | |
| 107 | $CH_3$ | H | H | H | $NH_2$ | |
| 108 | $CH_3$ | $NH-\overset{O}{\overset{\|}{C}}-CH_3$ | H | H | H | |
| 109 | $CH_3$ | H | H | H | $NH\overset{O}{\overset{\|}{C}}-CH_3$ | |
| 110 | $CH_3$ | CN | H | H | H | |
| 111 | $CH_3$ | H | CN | H | | |
| 112 | $CH_3$ | H | H | H | CN | |
| 113 | $CH_3$ | $C_6H_5$ | H | H | H | |
| 114 | $CH_3$ | H | $C_6H_5$ | H | H | 185 |
| 115 | $CH_3$ | H | H | $C_6H_5$ | H | |
| 116 | $CH_3$ | H | H | H | $C_6H_5$ | |
| 117 | $CH_3$ | $COOCH_3$ | H | H | H | |
| 118 | $CH_3$ | H | H | H | $COOCH_3$ | |
| 119 | $CH_3$ | COOH | H | H | H | |
| 120 | $CH_3$ | H | H | H | COOH | |
| 121 | $CH_3$ | $CONHCH_3$ | H | H | H | |
| 122 | $CH_3$ | H | H | H | $CONHCH_3$ | |
| 123 | $CH_3$ | $CON(CH_3)_2$ | H | H | H | |

| Beispiel Nr. | $R^1$ | $R^2$ | $R^3$ | $R^4$ | $R^5$ | Schmelzpunkt [oC] |
|---|---|---|---|---|---|---|
| 124 | $CH_3$ | H | H | H | $CON(CH_3)_2$ | |
| 125 | $C_6H_5$ | OH | H | H | OH | 208 |
| 126 | $C_6H_5$ | OH | H | H | H | |
| 127 | $C_6H_5$ | H | H | H | OH | |
| 128 | $C_6H_5$ | F | H | H | H | |
| 129 | $C_6H_5$ | H | H | F | H | |
| 130 | $C_6H_5$ | H | H | H | F | |
| 131 | $C_6H_5$ | Cl | H | H | H | |
| 132 | $C_6H_5$ | H | H | H | Cl | |
| 133 | $C_6H_5$ | OH | H | $OCH_3$ | H | |
| 134 | $C_6H_5$ | $OCH_3$ | H | OH | H | |
| 135 | $C_6H_5$ | H | $OCH_3$ | H | OH | |
| 136 | $C_6H_5$ | $CF_3$ | H | H | H | |
| 137 | $C_6H_5$ | H | H | $CF_3$ | H | |
| 138 | $C_6H_5$ | H | H | H | $CF_3$ | |
| 139 | $C_6H5$ | $NH_2$ | H | H | H | |
| 140 | $C_6H5$ | H | H | $NH_2$ | H | |
| 141 | $C_6H5$ | H | H | H | $NH_2$ | |
| 142 | $C_6H5$ | H | $NO_2$ | H | H | |
| 143 | $C_6H5$ | $NO_2$ | H | H | H | |
| 144 | $C_6H5$ | F | F | H | H | |

| Beispiel Nr. | $R^1$ | $R^2$ | $R^3$ | $R^4$ | $R^5$ | Schmelzpunkt [oC] |
|---|---|---|---|---|---|---|
| 145 | i-Propyl | H | $CF_3$ | H | H | |
| 146 | i-Propyl | H | H | $CF_3$ | H | |
| 147 | i-Propyl | H | H | H | $CF_3$ | |
| 148 | i-Propyl | H | $CH_3$ | H | OH | |
| 149 | i-Propyl | OH | H | $CH_3$ | H | |
| 150 | i-Propyl | H | $C_4H_4$ | | OH | |
| 151 | i-Propyl | H | $C_4H_4$ | | H | |
| 152 | i-Propyl | OH | $C_4H_4$ | | H | |
| 153 | i-Propyl | H | $C_4H_4$ | | $O-\overset{\overset{O}{\|}}{C}-CH_3$ | |
| 154 | i-Propyl | $O-\overset{\overset{O}{\|}}{C}-CH_3$ | $C_4H_4$ | | H | |
| 155 | i-Propyl | F | $C_4H_4$ | | OH | |
| 156 | i-Propyl | $NO_2$ | H | H | H | |
| 157 | i-Propyl | H | H | H | $NO_2$ | |
| 158 | i-Propyl | $NH_2$ | H | H | H | |
| 159 | i-Propyl | H | H | H | $NH_2$ | |
| 160 | i-Propyl | $NH-\overset{\overset{O}{\|}}{C}-CH_3$ | H | H | H | |
| 161 | i-Propyl | H | H | H | $NHCO-CH_3$ | |
| 162 | i-Propyl | CN | H | H | H | |

| Beispiel Nr. | R1 | R2 | R3 | R4 | R5 | Schmelzpunkt [oC] |
|---|---|---|---|---|---|---|
| 163 | i-Propyl | H | CN | H | H | |
| 164 | i-Propyl | H | H | H | CN | |
| 165 | i-Propyl | $C_6H_5$ | H | H | H | |
| 166 | i-Propyl | H | $C_6H_5$ | H | H | |
| 167 | i-Propyl | H | H | $C_6H_5$ | H | |
| 168 | i-Propyl | H | H | H | $C_6H_5$ | |
| 169 | i-Propyl | $COOCH_3$ | H | H | H | |
| 170 | i-Propyl | H | H | H | $COOCH_3$ | |
| 171 | i-Propyl | F | H | H | H | |
| 172 | i-Propyl | H | F | H | H | |
| 173 | i-Propyl | H | H | F | H | |
| 174 | i-Propyl | H | H | H | F | |
| 175 | i-Propyl | Cl | H | H | H | |
| 176 | i-Propyl | H | Cl | H | H | |
| 177 | i-Propyl | H | H | Cl | H | |
| 178 | i-Propyl | H | H | H | Cl | |
| 179 | i-Propyl | Br | H | H | H | |
| 180 | i-Propyl | F | H | H | Br | |
| 181 | i-Propyl | Cl | H | H | Cl | |
| 182 | i-Propyl | H | Cl | H | Cl | |
| 183 | i-Propyl | H | Cl | Cl | H | |
| 184 | i-Propyl | F | H | H | F | |

| Beispiel Nr. | $R^1$ | $R^2$ | $R^3$ | $R^4$ | $R^5$ | Schmelzpunkt [oC] |
|---|---|---|---|---|---|---|
| 185 | i-Propyl | H | F | H | F | |
| 186 | i-Propyl | H | F | F | H | |
| 187 | i-Propyl | OH | H | H | H | |
| 188 | i-Propyl | H | OH | H | H | |
| 189 | i-Propyl | H | H | OH | H | |
| 190 | i-Propyl | H | H | H | OH | |
| 191 | i-Propyl | OH | H | H | OH | |
| 192 | i-Propyl | $OCH_3$ | H | H | H | |
| 193 | i-Propyl | H | $OCH_3$ | H | H | 176 |
| 194 | i-Propyl | H | H | $OCH_3$ | H | 160 |
| 195 | i-Propyl | H | H | H | $OCH_3$ | |
| 196 | i-Propyl | $OCH_3$ | H | H | $OCH_3$ | |
| 197 | i-Propyl | OH | H | H | $OCH_3$ | |
| 198 | i-Propyl | $OCH_3$ | H | H | OH | |
| 199 | i-Propyl | $OCH_3$ | $OCH_3$ | H | $OCH_3$ | |
| 200 | i-Propyl | OH | $OCH_3$ | H | $OCH_3$ | |
| 201 | i-Propyl | $OCH_3$ | H | $OCH_3$ | $OCH_3$ | |
| 202 | i-Propyl | $OCH_3$ | H | $OCH_3$ | OH | |
| 203 | i-Propyl | OH | H | $OCH_3$ | H | |
| 204 | i-Propyl | H | $OCH_3$ | H | $OCH_3$ | |
| 205 | i-Propyl | H | $OCH_3$ | H | OH | |

| Beispiel Nr. | $R^1$ | $R^2$ | $R^3$ | $R^4$ | $R^5$ | Schmelzpunkt [oC] |
|---|---|---|---|---|---|---|
| 206 | i-Propyl | H | | $O-CH_2-CH_2O$ | H | |
| 207 | i-Propyl | H | | $O-CH_2-O$ | H | |
| 208 | i-Propyl | H | OH | OH | H | |
| 209 | i-Propyl | $S-CH_3$ | H | H | H | |
| 210 | i-Propyl | H | $S-CH_3$ | H | H | |
| 211 | i-Propyl | H | H | $SCH_3$ | H | |
| 212 | i-Propyl | H | H | H | $SCH_3$ | |
| 213 | i-Propyl | $O-\overset{\overset{O}{\|}}{C}-CH_3$ | H | H | H | |
| 214 | i-Propyl | H | H | H | $O-\overset{\overset{O}{\|}}{C}-CH_3$ | |
| 215 | i-Propyl | $CH_3$ | H | H | H | |
| 216 | i-Propyl | H | $CH_3$ | H | H | |
| 217 | i-Propyl | H | H | $CH_3$ | H | |
| 218 | i-Propyl | H | H | H | $CH_3$ | |
| 219 | i-Propyl | $CH_3$ | H | H | $CH_3$ | |
| 220 | i-Propyl | $CF_3$ | H | H | H | |
| 221 | i-Propyl | COOH | H | H | H | |
| 222 | i-Propyl | H | H | H | COOH | |
| 223 | i-Propyl | $CONHCH_3$ | H | H | H | |
| 224 | i-Propyl | H | H | H | $CONHCH_3$ | |
| 225 | i-Propyl | $CON(CH_3)_2$ | H | H | H | |

| Beispiel Nr. | $R^1$ | $R^2$ | $R^3$ | $R^4$ | $R^5$ | Schmelzpunkt [oC] |
|---|---|---|---|---|---|---|
| 226 | i-Propyl | H | H | H | $CON(CH_3)_2$ | |
| 227 | $CH_2-C\equiv CH$ | OH | H | H | H | |
| 228 | $CH_2-C\equiv CH$ | H | H | H | OH | |
| 229 | $CH_2-C\equiv CH$ | $OCH_3$ | H | H | H | |
| 230 | $CH_2-C\equiv CH$ | H | H | H | $OCH_3$ | |
| 231 | $CH_2-C\equiv CH$ | OH | H | $OCH_3$ | H | |
| 232 | $CH_2-C\equiv CH$ | H | $OCH_3$ | H | OH | |
| 233 | $CH_2-C\equiv CH$ | F | H | H | H | |
| 234 | $CH_2-C\equiv CH$ | H | H | F | H | |
| 235 | $CH_2-C\equiv CH$ | H | H | H | F | |
| 236 | $CH_2-C\equiv CH$ | Cl | H | H | H | |
| 237 | $CH_2-C\equiv CH$ | H | H | H | Cl | |
| 238 | $CH_2-C\equiv CH$ | H | H | H | Br | |
| 239 | $CH_2-C\equiv CH$ | $CF_3$ | H | H | H | |
| 240 | $CH_2-C\equiv CH$ | H | H | $CF_3$ | H | |
| 241 | $CH_2-C\equiv CH$ | H | H | H | $CF_3$ | |
| 242 | $CH_2-C\equiv CH$ | H | $OCH_3$ | H | H | |
| 243 | $CH_2-C\equiv CH$ | H | H | $OCH_3$ | H | |
| 244 | $CH_2-C\equiv CH$ | CN | H | H | H | |
| 245 | $CH_2-C\equiv CH$ | H | H | CN | H | |
| 246 | $CH_2-C\equiv CH$ | H | H | H | CN | |

| Beispiel Nr. | R1 | R2 | R3 | R4 | R5 | Schmelzpunkt [oC] |
|---|---|---|---|---|---|---|
| 247 | Allyl | F | H | H | H | |
| 248 | Allyl | H | F | H | H | |
| 249 | Allyl | H | H | F | H | |
| 250 | Allyl | H | H | H | F | |
| 251 | Allyl | Cl | H | H | H | |
| 252 | Allyl | H | Cl | H | H | |
| 253 | Allyl | H | H | Cl | H | |
| 254 | Allyl | H | H | H | Cl | |
| 255 | Allyl | Br | H | H | H | |
| 256 | Allyl | H | H | H | Br | |
| 257 | Allyl | Cl | H | H | Cl | |
| 258 | Allyl | H | Cl | H | Cl | |
| 259 | Allyl | H | Cl | Cl | H | |
| 260 | Allyl | F | H | H | F | |
| 261 | Allyl | H | F | H | F | |
| 262 | Allyl | H | F | F | H | |
| 263 | Allyl | OH | H | H | H | |
| 264 | Allyl | H | OH | H | H | |
| 265 | Allyl | H | H | OH | H | |
| 266 | Allyl | H | H | H | OH | |
| 267 | Allyl | OH | H | H | OH | |
| 268 | Allyl | $OCH_3$ | H | H | H | |

| Beispiel Nr. | $R^1$ | $R^2$ | $R^3$ | $R^4$ | $R^5$ | Schmelzpunkt [oC] |
|---|---|---|---|---|---|---|
| 269 | Allyl | H | $OCH_3$ | H | H | |
| 270 | Allyl | H | H | $OCH_3$ | H | |
| 271 | Allyl | H | H | H | H | |
| 272 | Allyl | $OCH_3$ | H | H | $OCH_3$ | |
| 273 | Allyl | OH | H | H | $OCH_3$ | |
| 274 | Allyl | $OCH_3$ | H | H | OH | |
| 275 | Allyl | $OCH_3$ | $OCH_3$ | H | $OCH_3$ | |
| 276 | Allyl | OH | $OCH_3$ | H | $OCH_3$ | |
| 277 | Allyl | $OCH_3$ | H | $OCH_3$ | $OCH_3$ | |
| 278 | Allyl | $OCH_3$ | H | $OCH_3$ | OH | |
| 279 | Allyl | OH | H | $OCH_3$ | H | |
| 280 | Allyl | H | $OCH_3$ | H | $OCH_3$ | |
| 281 | Allyl | H | $OCH_3$ | H | OH | |
| 282 | Allyl | H | $OCH_2-CH_2O$ | | H | |
| 283 | Allyl | H | $O-CH_2-O$ | | H | |
| 284 | Allyl | H | OH | OH | H | |
| 285 | Allyl | $S-CH_3$ | H | H | H | |
| 286 | Allyl | H | $S-CH_3$ | H | H | |
| 287 | Allyl | H | H | $S-CH_3$ | H | |
| 288 | Allyl | H | H | H | $SCH_3$ | |
| 289 | Allyl | $O-\overset{O}{\overset{\parallel}{C}}-CH_3$ | H | H | H | |

| Beispiel Nr. | $R^1$ | $R^2$ | $R^3$ | $R^4$ | $R^5$ | Schmelzpunkt [oC] |
|---|---|---|---|---|---|---|
| 290 | Allyl | H | H | H | $O-\overset{O}{\overset{\|\|}{C}}-CH_3$ | |
| 291 | Allyl | $CH_3$ | H | H | H | |
| 292 | Allyl | H | $CH_3$ | H | H | |
| 293 | Allyl | H | H | $CH_3$ | H | |
| 294 | Allyl | H | H | H | H | |
| 295 | Allyl | $CH_3$ | H | H | $CH_3$ | |
| 296 | Allyl | $CF_3$ | H | H | H | |
| 297 | Allyl | H | $CF_3$ | H | H | |
| 298 | Allyl | H | H | $CF_3$ | H | |
| 299 | Allyl | H | H | H | $CF_3$ | |
| 300 | Allyl | H | $CH_3$ | H | OH | |
| 301 | Allyl | OH | H | $CH_3$ | H | |
| 302 | Allyl | H | $C_4H_4$ | | OH | |
| 303 | Allyl | H | $C_4H_4$ | | H | |
| 304 | Allyl | OH | $C_4H_4$ | | H | |
| 305 | Allyl | H | $C_4H_4$ | | $O-\overset{O}{\overset{\|\|}{C}}-CH_3$ | |
| 306 | Allyl | $O-\overset{O}{\overset{\|\|}{C}}-CH_3$ | $C_4H_4$ | | H | |
| 307 | Allyl | F | $C_4H_4$ | | OH | |
| 308 | Allyl | $NO_2$ | H | H | H | |

| Beispiel Nr. | $R^1$ | $R^2$ | $R^3$ | $R^4$ | $R^5$ | Schmelzpunkt [oC] |
|---|---|---|---|---|---|---|
| 309 | Allyl | H | H | H | $NO_2$ | |
| 310 | Allyl | $NH_2$ | H | H | H | |
| 311 | Allyl | H | H | H | $NH_2$ | |
| 312 | Allyl | $NH-C(=O)-CH_3$ | H | H | H | |
| 313 | Allyl | H | H | H | $NHC(=O)-CH_3$ | |
| 314 | Allyl | CN | H | H | H | |
| 315 | Allyl | H | CN | H | H | |
| 316 | Allyl | H | H | H | CN | |
| 317 | Allyl | $C_6H_5$ | H | H | H | |
| 318 | Allyl | H | $C_6H_5$ | H | H | |
| 319 | Allyl | H | H | $C_6H_5$ | H | |
| 320 | Allyl | H | H | H | $C_6H_5$ | |
| 321 | Allyl | $COOCH_3$ | H | H | H | |
| 322 | Allyl | H | H | H | $COOCH_3$ | |
| 323 | Allyl | COOH | H | H | H | |
| 324 | Allyl | H | H | H | COOH | |
| 325 | Allyl | $CONHCH_3$ | H | H | H | |
| 326 | Allyl | H | H | H | $CONHCH_3$ | |
| 327 | Allyl | $CON(CH_3)_2$ | H | H | H | |
| 328 | Allyl | H | H | H | $CON(CH_3)2$ | |

| Beispiel Nr. | R1 | R2 | R3 | R4 | R5 | Schmelzpunkt [oC] |
|---|---|---|---|---|---|---|
| 329 | Allyl | $N(CH_3)_2$ | H | H | H | |
| 330 | Allyl | H | H | H | $N(CH_3)2$ | |
| 331 | Allyl | F | H | H | $NH_2$ | |
| 332 | Allyl | $NH_2$ | H | H | F | |

Die Wirkstoffe der herbiziden Mittel sind herbizid wirksam und gegenüber Kulturpflanzen selektiv.

Die Verbindungen können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz

nach den Verwendungszwecken; sie sollten in jedem Fall möglichst die feinste Verteilung der erfindungsgemäßen Wirkstoffe gewährleisten.

Die Verbindungen eignen sich allgemein zur Herstellung von direkt versprühbaren Lösungen, Emulsionen, Pasten oder Öldispersionen aus Mineralölfraktionen von mittlerem bis hohem Siedepunkt, wie Kerosin oder Dieselöl, ferner aus Kohlenteerölen sowie aus Ölen pflanzlichen oder tierischen Ursprungs, aus aliphatischen, cyclischen und aromatischen Kohlenwasserstoffen, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron, oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali- und Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 2 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 4 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung N. 6 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 59 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 45 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle

vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 4 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 6 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 59 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0,01 bis 5,0, vorzugsweise 0,05 bis 3,0 kg/ha.

Die herbizide Wirkung der Benzindazole der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt eingesät.

Zum Zweck der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,5 bzw. 1,0 kg/ha a:S.

Die Versuchsgefäße werden im Gewächshaus aufgestellt, wobei für wärmeliebende Arten wärmere Bereiche (20 bis 35 ° C) und für solche gemäßigter Klimate 10 bis 20 ° C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Amaranthus retroflexus | Zurückgekrümmter Fuchsschwanz |
| Ipomoea spp. | Prunkwindearten |
| Setaria italica | Ital. Raygras |
| Solanum nigrum | Schwarzer Nachtschatten |
| Veronica spp. | Ehrenpreisarten |

Mit 0,5 bzw. 1,0 kg Wirkstoff/ha im Nachauflaufverfahren eingesetzt, lassen sich mit den Verbindungen aus den Beispielen 2, 4, 6, 45 und 59 unerwünschte Pflanzen sehr gut bekämpfen.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |

| Botanischer Name | Deutscher Name |
|---|---|
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |

| Botanischer Name | Deutscher Name |
|---|---|
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Naphthindazole der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäuren, (Het)Aryloxy-phenoxy-propionsäuren, deren Salze, Ester und Amide und andere in Betracht.

Außerdem kann es von Nutzen sein, die neuen Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopatogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

### Patentansprüche für folgende Vertragsstaaten : CH, DE, FR, GB, IT, LI, NL

**1.** Benzindazol-4,9-chinone der Formel

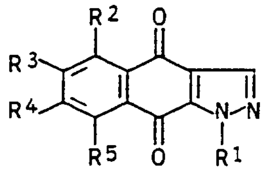

(I),

in der

$R^1$ $C_2$-$C_{10}$-Alkyl, $C_2$-$C_{10}$-Alkenyl, $C_2$-$C_{10}$-Alkinyl, $C_1$-$C_{10}$-Halogenalkyl, $C_1$-$C_{10}$-Hydroxyalkyl, $C_2$-$C_{14}$-Alkoxyalkyl, $C_2$-$C_{14}$-Alkylthioalkyl, $C_3$-$C_7$-Cycloalkyl, $C_1$-$C_4$-Alkylcarbonyloxy-$C_1$-$C_4$-alkyl, $C_1$-$C_4$-Alkylaminocarbonyloxy-$C_1$-$C_4$-alkyl, gegebenenfalls durch Halogen, $C_1$-$C_4$-Alkyl oder $C_1$-$C_4$-Alkoxy im Phenylrest substituiertes Phenylaminocarbonyloxy-$C_1$-$C_4$-alkyl, gegebenenfalls durch Ha-

logen, $C_1$-$C_4$-Alkyl oder $C_1$-$C_4$-Alkoxy im Phenylrest substituiertes Phenylsulfonyloxy-$C_1$-$C_4$-Alkyl, $C_1$-$C_4$-Alkoxycarbonyl-$C_1$-$C_4$-alkyl, Hydroxycarbonyl-$C_1$-$C_4$-alkyl, Aminocarbonyl-$C_1$-$C_4$-alkyl, $C_1$-$C_4$-Alkylaminocarbonyl-$C_1$-$C_4$-alkyl, Di-($C_1$-$C_4$-Alkyl)-aminocarbonyl-$C_1$-$C_4$-alkyl, Halogen-$C_3$-$C_4$-alkinyl, einen gegebenenfalls durch Halogen oder $C_1$-$C_4$-Alkyl substituierten Heteroarylrest mit einem oder zwei Stickstoffatomen, wie Pyrimidyl, Pyridyl, Imidazolyl, einen gegebenenfalls durch Halogen, Hydroxy, Nitro, Amino, $C_1$-$C_4$-Alkylamino, Di-$C_1$-$C_4$-alkylamino, Cyano, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Alkylthio oder $C_1$-$C_4$-Halogenalkylthio substituierten Phenylrest oder einen gegebenenfalls durch Halogen substituierten Benzylrest und

$R^2$, $R^3$, $R^4$ und $R^5$ unabhängig voneinander Wasserstoff, Halogen, Nitro, Cyano, Hydroxy, Amino, $C_1$-$C_4$-Alkylamino, Di-$C_1$-$C_4$-alkylamino, $C_1$-$C_6$-Alkyl, $C_1$-$C_5$-Halogenalkyl, $C_1$-$C_5$-Alkoxy, $C_1$-$C_5$-Halogenalkoxy, $C_1$-$C_5$-Alkylthio, $C_1$-$C_5$-Halogenalkylthio, $C_2$-$C_{10}$-Alkoxyalkyl, Carboxyl, $C_2$-$C_6$-Alkoxycarbonyl, $C_2$-$C_6$-Alkanoyloxy, $C_2$-$C_6$-Halogenalkanoyloxy, $C_1$-$C_4$-Alkylcarbonylamino, $C_1$-$C_4$-Alkylaminocarbonyl, Di-($C_1$-$C_4$-Alkyl)-aminocarbonyl, oder einen gegebenenfalls durch Halogen, Trifluormethyl, Nitro, Cyano, Amino, $C_1$-$C_4$-Alkoxy, $C_1$-$C_4$-Alkylthio oder $C_1$-$C_5$-Halogenalkylthio substituierten Phenyl- oder Heteroarylrest wie Pyridyl, Thienyl, Furyl, Benzimidazol-2-yl oder Pyrimid-2-yl, bedeuten

und außerdem

$R^3$ und $R^4$ gemeinsam mit den beiden Kohlenstoffatomen des Phenylringes, an die sie gebunden sind, einen gegebenenfalls durch Halogen, Nitro, Cyano, Amino, Hydroxy, Trifluormethyl, $C_1$-$C_5$-Alkyl, $C_1$-$C_5$-Alkoxy, $C_1$-$C_5$-Halogenalkylthio, $C_1$-$C_5$-Halogenalkoxy oder $C_1$-$C_5$-Alkylthio substituierten Benzol- oder Naphthalinring bilden oder einen heterocyclischen Ring, ausgewählt aus der Gruppe 1,4-Dioxan, 1,3-Dioxolan, Pyrazol, Indol, Thiophen, Triazol und Piperazin, 2,2-Dimethyl-1,3-dioxolan, 1-Methylpyrazol, 1-Methyl-indol, N,N'-Dimethylpiperazin, 2,2-Diphenyl-1,3-dioxolan, 2-Oxo-1,3-dioxolan, ausgenommen 1-Vinyl-benzindazol-4,9-chinon.

2. Benzindazol-4,9-chinone der Formel I gemäß Anspruch 1, dadurch gekennzeichnet, daß $R^1$ $C_2$-$C_4$-Alkyl oder $C_2$-$C_4$-Alkenyl bedeutet.

3. Benzindazol-4,9-chinone der Formel I gemäß Anspruch 1, dadurch gekennzeichnet, daß $R^2$ bis $R^5$ unabhängig voneinander Wasserstoff, Hydroxy oder Halogen bedeuten.

4. Verfahren zur Herstellung von Benzindazol-4,9-chinonen der Formel I gemäß Anspruch 1, dadurch gekennzeichnet, daß man 5-Arylmethylpyrazol-4-carbonsäurehalogenide der Formel

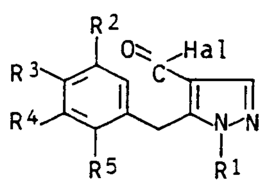

(II),

in der $R^1$, $R^2$, $R^3$, $R^4$ und $R^5$ die im Anspruch 1 angegebenen Bedeutungen haben und Hal für Halogen steht, in einem inerten Lösungsvermittler in Gegenwart eines Friedel-Crafts-Katalysators zur Reaktion bringt und die entstehenden Verbindungen der Formel

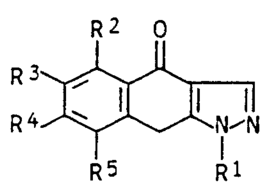

(III),

in der $R^1$, $R^2$, $R^3$, $R^4$ und $R^5$ die oben angegebenen Bedeutungen haben, in Gegenwart eines Verdünnungsmittels mit Oxidationsmitteln in an sich üblicher Weise in die Benzindazol-4,9-chinone nach Formel I überführt.

**5.** Herbizides Mittel, enthaltend ein Benzindazol-4,9-chinon der Formel

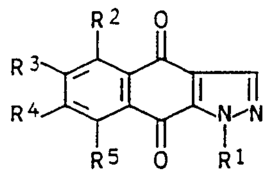

(Ia),

in der
$R^1$, $R^2$, $R^3$, $R^4$ und $R^5$ die für Formel I in Anspruch 1 angegebenen Bedeutungen haben und $R^1$ zusätzlich für Wasserstoff oder Methyl stehen kann sowie ferner für Vinyl auch wenn $R^2$ bis $R^5$ Wasserstoff bedeuten.

**6.** Herbizides Mittel gemäß Anspruch 5, dadurch gekennzeichnet, daß es Benzindazol-4,9-chinone der Formel Ia enthält, in der $R^1$ $C_1$-$C_4$-Alkyl oder $C_2$-$C_4$-Alkenyl bedeutet.

**7.** Herbizides Mittel gemäß Anspruch 5, dadurch gekennzeichnet, daß es Benzindazol-4,9-chinone der Formel Ia enthält, in der $R^2$ bis $R^5$ unabhängig voneinander Wasserstoff, Hydroxy, Halogen, Nitro, Cyano, Amino, $C_1$-$C_4$-Alkylamino oder Di-$C_1$-$C_4$-alkylamino bedeuten.

**8.** Herbizides Mittel, enthaltend inerte Zusatzstoffe und ein Benzindazol-4,9-chinon der Formel Ia gemäß Anspruch 5.

**9.** Verfahren zur Bekämpfung unerwünschten Pflanzenwachstums, dadurch gekennzeichnet, daß man ein Benzindazol-4,9-chinon der Formel Ia gemäß Anspruch 5 in einer herbizid wirksamen Menge auf die Pflanzen und/oder ihren Lebensraum einwirken läßt.

**Patentansprüche für folgenden Vertragsstaat : ES**

**1.** Verfahren zur Herstellung von Benzindazol-4,9-chinonen der Formel

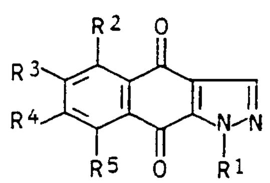

(I),

in der

$R^1$ $C_2$-$C_{10}$-Alkyl, $C_2$-$C_{10}$-Alkenyl, $C_2$-$C_{10}$-Alkinyl, $C_1$-$C_{10}$-Halogenalkyl, $C_1$-$C_{10}$-Hydroxyalkyl, $C_2$-$C_{14}$-Alkoxyalkyl, $C_2$-$C_{14}$-Alkylthioalkyl, $C_3$-$C_7$-Cycloalkyl, $C_1$-$C_4$-Alkylcarbonyloxy-$C_1$-$C_4$-alkyl, $C_1$-$C_4$-Alkylaminocarbonyloxy-$C_1$-$C_4$-alkyl, gegebenenfalls durch Halogen, $C_1$-$C_4$-Alkyl oder $C_1$-$C_4$-Alkoxy im Phenylrest substituiertes Phenylaminocarbonyloxy-$C_1$-$C_4$-alkyl, gegebenenfalls durch Halogen, $C_1$-$C_4$-Alkyl oder $C_1$-$C_4$-Alkoxy im Phenylrest substituiertes Phenylsulfonyloxy-$C_1$-$C_4$-Alkyl, $C_1$-$C_4$-Alkoxycarbonyl-$C_1$-$C_4$-alkyl, Hydroxycarbonyl-$C_1$-$C_4$-alkyl, Aminocarbonyl-$C_1$-$C_4$-alkyl, $C_1$-$C_4$-Alkylaminocarbonyl-$C_1$-$C_4$-alkyl, Di-($C_1$-$C_4$-Alkyl)-aminocarbonyl-$C_1$-$C_4$-alkyl, Halogen-$C_3$-$C_4$-alkinyl, einen gegebenenfalls durch Halogen oder $C_1$-$C_4$-Alkyl substituierten Heteroarylrest mit einem oder zwei Stickstoffatomen wie Pyrimidyl, Pyridyl, Imidazolyl, einen gegebenenfalls durch Halogen, Hydroxy, Nitro, Amino, $C_1$-$C_4$-Alkylamino, Di-

$C_1$-$C_4$-alkylamino, Cyano, $C_1$-$C_4$-Alkyl, $C_1$-$C_4$-Alxoxy, $C_1$-$C_4$-Halogenalkoxy, $C_1$-$C_4$-Halogenalkyl, $C_1$-$C_4$-Alkylthio oder $C_1$-$C_4$-Halogenalkylthio substituierten Phenylrest oder einen gegebenenfalls durch Halogen substituierten Benzylrest und

$R^2$, $R^3$, $R^4$ und $R^5$ unabhängig voneinander Wasserstoff, Halogen, Nitro, Cyano, Hydroxy, Amino, $C_1$-$C_4$-Alkylamino, Di-$C_1$-$C_4$-alkylamino, $C_1$-$C_6$-Alkyl, $C_1$-$C_5$-Halogenalkyl, $C_1$-$C_5$-Alkoxy, $C_1$-$C_5$-Halogenalkoxy, $C_1$-$C_5$-Alkylthio, $C_1$-$C_5$-Halogenalkylthio, $C_2$-$C_{10}$-Alkoxyalkyl, Carboxyl, $C_2$-$C_6$-Alkoxycarbonyl, $C_2$-$C_6$-Alkanoyloxy, $C_2$-$C_6$-Halogenalkanoyloxy, $C_1$-$C_4$-Alkylcarbonylamino, $C_1$-$C_4$-Alkylaminocarbonyl, Di-($C_1$-$C_4$-Alkyl)-aminocarbonyl, oder einen gegebenenfalls durch Halogen, Trifluormethyl, Nitro, Cyano, Amino, $C_1$-$C_4$-Alkoxy, $C_1$-$C_4$-Alkylthio oder $C_1$-$C_5$-Halogenalkylthio substituierten Phenyl- oder Heteroarylrest wie Pyridyl, Thienyl, Furyl, Benzimidazol-2-yl oder Pyrimid-2-yl, bedeuten

und außerdem

$R^3$ und $R^4$ gemeinsam mit den beiden Kohlenstoffatomen des Phenylringes, an die sie gebunden sind, einen heterocyclischen Ring ausgewählt aus der Gruppe 1,4-Dioxan, 1,3-Dioxolan, Pyrazol, Indol, Thiophen, Triazol und Piperazin, 2,2-Dimethyl-1,3-dioxalan, 1-Methylpyrazol, 1-Methyl-indol, N,N'-Dimethylpiperazin, 2,2-Diphenyl-1,3-dioxolan, 2-Oxo-1,3-dioxolan oder einen gegebenenfalls durch Halogen, Nitro, Cyano, Amino, Hydroxy, Trifluormethyl, $C_1$-$C_5$-Alkyl, $C_1$-$C_5$-Alkoxy, $C_1$-$C_5$-Halogenalkylthio, $C_1$-$C_5$-Halogenalkoxy oder $C_1$-$C_5$-Alkylthio substituierten Benzol- oder Naphthalinring bilden, ausgenommen 1-Vinylbenzindazol-4,9-chinon, dadurch gekennzeichnet, daß man 5-Arylmethylpyrazol-4-carbonsäurehalogenide der Formel

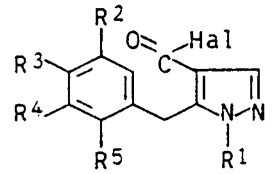

(II),

in der $R^1$, $R^2$, $R^3$, $R^4$ und $R^5$ die oben angegebenen Bedeutungen haben und Hal für Halogen steht, in einem inerten Lösungsvermittler in Gegenwart eines Friedel-Crafts-Katalysators zur Reaktion bringt und die entstehenden Verbindungen der Formel

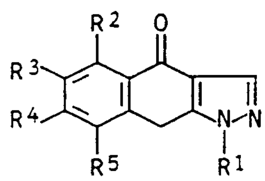

(III),

in der $R^1$, $R^2$, $R^3$, $R^4$ und $R^5$ die oben angegebenen Bedeutungen haben, in Gegenwart eines Verdünnungsmittels mit Oxidationsmitteln in an sich üblicher Weise in die Benzindazol-4,9-chinone nach Formel I überführt.

**2.** Herbizides Mittel, enthaltend ein Benzindazol-4,9-chinon der Formel

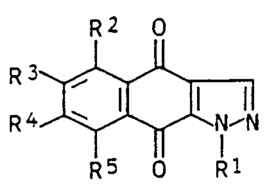

(Ia),

in der

$R^1$, $R^2$, $R^3$, $R^4$ und $R^5$ die für Formel I in Anspruch 1 angegebenen Bedeutungen haben und $R^1$

zusätzlich für Wasserstoff oder Methyl stehen kann sowie ferner für Vinyl, auch wenn $R^2$ bis $R^5$ Wasserstoff bedeuten.

**3.** Verfahren zur Bekämpfung unerwünschten Pflanzenwachstums, dadurch gekennzeichnet, daß man ein Benzindazol-4,9-chinon der Formel Ia gemäß Anspruch 2 in einer herbizid wirksamen Menge auf die Pflanzen und/oder ihren Lebensraum einwirken läßt.

**Claims**

**Claims for the following Contracting States : CH, DE, FR, GB, IT, LI, NL**

**1.** Benzindazole-4,9-quinones of the formula

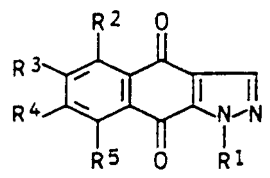

(I),

where:

$R^1$ is $C_2$-$C_{10}$-alkyl, $C_2$-$C_{10}$-alkenyl, $C_2$-$C_{10}$-alkynyl, $C_1$-$C_{10}$-haloalkyl, $C_1$-$C_{10}$-hydroxyalkyl, $C_2$-$C_{14}$-alkoxyalkyl, $C_2$-$C_{14}$-alkylthioalkyl, $C_3$-$C_7$-cycloalkyl, $C_1$-$C_4$-alkylcarbonyloxy-$C_1$-$C_4$-alkyl, $C_1$-$C_4$-alkylaminocarbonyloxy-$C_1$-$C_4$-alkyl, phenylaminocarbonyloxy-$C_1$-$C_4$-alkyl which is unsubstituted or substituted in the phenyl moiety by halogen, $C_1$-$C_4$-alkyl or $C_1$-$C_4$-alkoxy, phenylsulfonyloxy-$C_1$-$C_4$-alkyl which is unsubstituted or substituted in the phenyl moiety by halogen, $C_1$-$C_4$-alkyl or $C_1$-$C_4$-alkoxy, $C_1$-$C_4$-alkoxycarbonyl-$C_1$-$C_4$-alkyl, hydroxycarbonyl-$C_1$-$C_4$-alkyl, aminocarbonyl-$C_1$-$C_4$-alkyl, $C_1$-$C_4$-alkylaminocarbonyl-$C_1$-$C_4$-alkyl, di-($C_1$-$C_4$-alkyl)-aminocarbonyl-$C_1$-$C_4$-alkyl, halo-$C_3$-$C_4$-alkynyl, an unsubstituted or halogen- or $C_1$-$C_4$-alkyl-substituted heteroaryl radical with one or two nitrogen atoms, such as pyrimidyl, pyridyl or imidazolyl, phenyl which is unsubstituted or substituted by halogen, hydroxyl, nitro, amino, $C_1$-$C_4$-alkylamino, di-$C_1$-$C_4$-alkylamino, cyano, $C_1$-$C_4$-alkyl, $C_1$-$C_4$-alkoxy, $C_1$-$C_4$-haloalkoxy, $C_1$-$C_4$-haloalkyl, $C_1$-$C_4$-alkylthio or $C_1$-$C_4$-haloalkylthio, or unsubstituted or halogen-substituted benzyl, and

$R^2$, $R^3$, $R^4$ and $R^5$ are independently of each other hydrogen, halogen, nitro, cyano, hydroxyl, amino, $C_1$-$C_4$-alkylamino, di-$C_1$-$C_4$-alkylamino, $C_1$-$C_6$-alkyl, $C_1$-$C_5$-haloalkyl, $C_1$-$C_5$-alkoxy, $C_1$-$C_5$-haloalkoxy, $C_1$-$C_5$-alkylthio, $C_1$-$C_5$-haloalkylthio, $C_2$-$C_{10}$-alkoxyalkyl, carboxyl, $C_2$-$C_6$-alkoxycarbonyl, $C_2$-$C_6$-alkanoyloxy, $C_2$-$C_6$-haloalkanoyloxy, $C_1$-$C_4$-alkylcarbonylamino, $C_1$-$C_4$-alkylaminocarbonyl, di-($C_1$-$C_4$-alkyl)-aminocarbonyl, or phenyl or heteroaryl, each of which is unsubstituted or substituted by halogen, trifluoromethyl, nitro, cyano, amino, $C_1$-$C_4$-alkoxy, $C_1$-$C_4$-alkylthio or $C_1$-$C_5$-haloalkylthio, such as pyridyl, thienyl, furyl, 2-benzimidazolyl or 2-pyrimidyl,

and furthermore

$R^3$ and $R^4$, together with the two carbon atoms of the phenyl ring to which they are attached, form a benzene or naphthalene ring system which is unsubstituted or substituted by halogen, nitro, cyano, amino, hydroxyl, trifluoromethyl, $C_1$-$C_5$-alkyl, $C_1$-$C_5$-alkoxy, $C_1$-$C_5$-haloalkylthio, $C_1$-$C_5$-haloalkoxy or $C_1$-$C_5$-alkylthio or a heterocyclic ring selected from the group consisting of 1,4-dioxane, 1,3-dioxolane, pyrazole, indole, thiophene, triazole and piperazine, 2,2-dimethyl-1,3-dioxolane, 1-methylpyrazole, 1-methylindole, N,N'-dimethylpiperazine, 2,2-diphenyl-1,3-dioxolane and 2-oxo-1,3-dioxolane, with the exception of 1-vinylbenzindazole-4,9-quinone.

**2.** Benzindazole-4,9-quinones of the formula I as set forth in claim 1 wherein $R^1$ is $C_2$-$C_4$-alkyl or $C_2$-$C_4$-alkenyl.

**3.** Benzindazole-4,9-quinones of the formula I as set forth in claim 1 wherein $R^2$ to $R^5$ are independently of each other hydrogen, hydroxyl or halogen.

**4.** A process for preparing benzindazole-4,9-quinones of the formula I as set forth in claim 1, which comprises reacting 5-arylmethylpyrazole-4-carbonyl halides of the formula

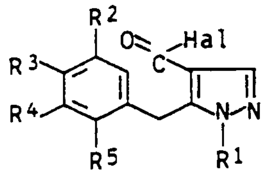

(II),

in which $R^1$, $R^2$, $R^3$, $R^4$ and $R^5$ are each as defined in claim 1 and Hal is halogen, in an inert solubilizer in the presence of a Friedel-Crafts catalyst and converting the resultant compounds of the formula

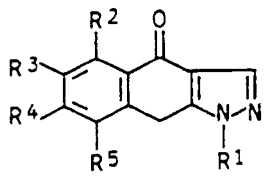

(III),

in which $R^1$, $R^2$, $R^3$, $R^4$ and $R^5$ are each as defined above, with oxidizing agents in the presence of a diluent in a conventional manner into the benzindazole-4,9-quinones of formula I.

**5.** A herbicidal composition comprising a benzindazole-4,9-quinone of the formula

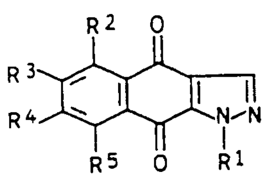

(Ia),

where
$R^1$, $R^2$, $R^3$, $R^4$ and $R^5$ are each as defined for the formula I in claim 1 and $R^1$ may additionally be hydrogen or methyl, and may also be vinyl, even when $R^2$ to $R^5$ are each hydrogen.

**6.** A herbicidal composition as set forth in claim 5 wherein the benzindazole-4,9-quinones are of the formula Ia where $R^1$ is $C_1$-$C_4$-alkyl or $C_2$-$C_4$-alkenyl.

**7.** A herbicidal composition as set forth in claim 5 wherein the benzindazole-4,9-quinones are of the formula Ia where $R^2$ to $R^5$ are independently of each other hydrogen, hydroxyl, halogen, nitro, cyano, amino, $C_1$-$C_4$-alkylamino or di-$C_1$-$C_4$-alkylamino.

**8.** A herbicidal composition comprising inert additives and a benzindazole-4,9-quinone of the formula Ia as set forth in claim 5.

**9.** A method of controlling unwanted plant growth, which comprises applying to the plants and/or their habitat a herbicidally effective amount of a benzindazole-4,9-quinone of the formula Ia as set forth in claim 5.

**Claims for the following Contracting State : ES**

**1.** A process for the preparation of benzindazole-4,9-quinones of the formula

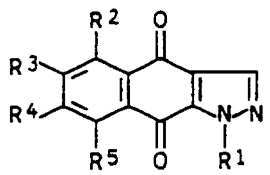

(I),

where:

$R^1$ is $C_2$-$C_{10}$-alkyl, $C_2$-$C_{10}$-alkenyl, $C_2$-$C_{10}$-alkynyl, $C_1$-$C_{10}$-haloalkyl, $C_1$-$C_{10}$-hydroxyalkyl, $C_2$-$C_{14}$-alkoxyalkyl, $C_2$-$C_{14}$-alkylthioalkyl, $C_3$-$C_7$-cycloalkyl, $C_1$-$C_4$-alkylcarbonyloxy-$C_1$-$C_4$-alkyl, $C_1$-$C_4$-alkylaminocarbonyloxy-$C_1$-$C_4$-alkyl, phenylaminocarbonyloxy-$C_1$-$C_4$-alkyl which is unsubstituted or substituted in the phenyl moiety by halogen, $C_1$-$C_4$-alkyl or $C_1$-$C_4$-alkoxy, phenylsulfonyloxy-$C_1$-$C_4$-alkyl which is unsubstituted or substituted in the phenyl moiety by halogen, $C_1$-$C_4$-alkyl or $C_1$-$C_4$-alkoxy, $C_1$-$C_4$-alkoxycarbonyl-$C_1$-$C_4$-alkyl, hydroxycarbonyl-$C_1$-$C_4$-alkyl, aminocarbonyl-$C_1$-$C_4$-alkyl, $C_1$-$C_4$-alkylaminocarbonyl-$C_1$-$C_4$-alkyl, di-($C_1$-$C_4$-alkyl)-aminocarbonyl-$C_1$-$C_4$-alkyl, halo-$C_3$-$C_4$-alkynyl, an unsubstituted or halogen- or $C_1$-$C_4$-alkyl-substituted heteroaryl radical with one or two nitrogen atoms, such as pyrimidyl, pyridyl or imidazolyl, phenyl which is unsubstituted or substituted by halogen, hydroxyl, nitro, amino, $C_1$-$C_4$-alkylamino, di-$C_1$-$C_4$-alkylamino, cyano, $C_1$-$C_4$-alkyl, $C_1$-$C_4$-alkoxy, $C_1$-$C_4$-haloalkoxy, $C_1$-$C_4$-haloalkyl, $C_1$-$C_4$-alkylthio or $C_1$-$C_4$-haloalkylthio, or unsubstituted or halogen-substituted benzyl, and

$R^2$, $R^3$, $R^4$ and $R^5$ are independently of each other hydrogen, halogen, nitro, cyano, hydroxyl, amino, $C_1$-$C_4$-alkylamino, di-$C_1$-$C_4$-alkylamino, $C_1$-$C_6$-alkyl, $C_1$-$C_5$-haloalkyl, $C_1$-$C_5$-alkoxy, $C_1$-$C_5$-haloalkoxy, $C_1$-$C_5$-alkylthio, $C_1$-$C_5$-haloalkylthio, $C_2$-$C_{10}$-alkoxyalkyl, carboxyl, $C_2$-$C_6$-alkoxycarbonyl, $C_2$-$C_6$-alkanoyloxy, $C_2$-$C_6$-haloalkanoyloxy, $C_1$-$C_4$-alkylcarbonylamino, $C_1$-$C_4$-alkylaminocarbonyl, di-($C_1$-$C_4$-alkyl)-aminocarbonyl, or phenyl or heteroaryl, each of which is unsubstituted or substituted by halogen, trifluoromethyl, nitro, cyano, amino, $C_1$-$C_4$-alkoxy, $C_1$-$C_4$-alkylthio or $C_1$-$C_5$-haloalkylthio, such as pyridyl, thienyl, furyl, 2-benzimidazolyl or 2-pyrimidyl,

and furthermore

$R^3$ and $R^4$, together with the two carbon atoms of the phenyl ring to which they are attached, form a heterocyclic ring selected from the group consisting of 1,4-dioxane, 1,3-dioxolane, pyrazole, indole, thiophene, triazole and piperazine, 2,2-dimethyl-1,3-dioxolane, 1-methyl-pyrazole, 1-methylindole, N,N'-dimethylpiperazine, 2,2-diphenyl-1,3-dioxolane and 2-oxo-1,3-dioxolane, or a benzene or naphthalene ring system which is unsubstituted or substituted by halogen, nitro, cyano, amino, hydroxyl, trifluoromethyl, $C_1$-$C_5$-alkyl, $C_1$-$C_5$-alkoxy, $C_1$-$C_5$-haloalkylthio, $C_1$-$C_5$-haloalkoxy or $C_1$-$C_5$-alkylthio, with the exception of 1-vinylbenzindazole-4,9-quinone, which comprises reacting 5-arylmethylpyrazole-4-carbonyl halides of the formula

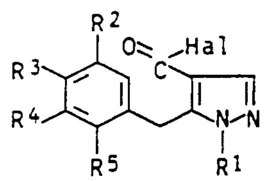

(II),

in which $R^1$, $R^2$, $R^3$, $R^4$ and $R^5$ are each as defined above and Hal is halogen, in an inert solubilizer in the presence of a Friedel-Crafts catalyst and converting the resultant compounds of the formula

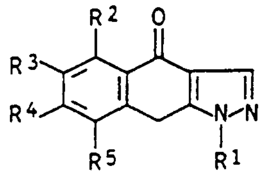

(III),

in which $R^1$, $R^2$, $R^3$, $R^4$ and $R^5$ are each as defined above, with oxidizing agents in the presence of a diluent in a conventional manner into the benzindazole-4,9-quinones of formula I.

**2.** A herbicidal composition comprising a benzindazole-4,9-quinone of the formula

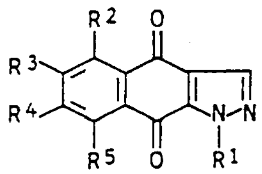

(Ia),

where
$R^1$, $R^2$, $R^3$, $R^4$ and $R^5$ are each as defined for the formula I in claim 1 and $R^1$ may additionally be hydrogen or methyl, and may also be vinyl, even when $R^2$ to $R^5$ are each hydrogen.

**3.** A method of controlling unwanted plant growth, which comprises applying to the plants and/or their habitat a herbicidally effective amount of a benzindazole-4,9-quinone of the formula Ia as set forth in claim 2.

**Revendications**

**Revendications pour les Etats contractants suivants : CH, DE, FR, GB, IT, LI, NL**

**1.** Benzoindazole-4,9-quinones de formule

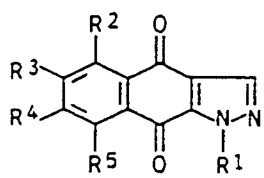

(I),

dans laquelle
$R^1$ représente un groupe alkyle en C2-C10, alcényle en C2-C10, alcynyle en C2-C10, halogénoalkyle en C1-C10, hydroxyalkyle en C1-C10, alcoxyalkyle en C2-C14, alkylthioalkyle en C2-C14, cycloalkyle en C3-C7, (alkyle en C1-C4)-carbonyloxy-alkyle en C1-C4, (alkyle en C1-C4)-aminocarbonyloxy-alkyle en C1-C4, phénylaminocarbonyloxy-alkyle en C1-C4 éventuellement substitué dans le groupe phényle par des halogènes, des groupes alkyle en C1-C4, ou alcoxy en C1-C4, phénylsulfonyloxy-alkyle en C1-C4 éventuellement substitué dans la partie phényle par des halogènes, des groupes alkyle en C1-C4 ou alcoxy en C1-C4, (alcoxy en C1-C4)-carbonyl-alkyle en C1-C4, hydroxycarbonyl-alkyle en C1-C4, aminocarbonyl-alkyle en C1-C4, (alkyle en C1-C4)-aminocarbonyl-alkyle en C1-C4, di-(alkyle en C1-C4)-aminocarbonyl-alkyle en C1-C4, halogénoalcynyle en C3-C4, un groupe hétéroaryle éventuellement substitué par des halogènes ou des groupes alkyle en C1-C4 et contenant 1 ou 2 atomes d'azote, tel que pyrimidyle, pyridyle, imidazolyle, un groupe phényle éventuellement substitué par des halogénes, des groupes hydroxy, nitro, amino, alkylamino en C1-C4, di-(alkyle en C1-C4)-amino, cyano, alkyle en C1-C4, alcoxy en C1-C4, halogénoalcoxy en C1-C4, halogénoalkyle en C1-C4, alkylthio en C1-C4 ou halogénoalkylthio en C1-C4, ou un groupe benzyle éventuellement substitué par des halogènes, et
$R^2$, $R^3$, $R^4$ et $R^5$ représentent chacun, indépendamment les uns des autres, l'hydrogène, un halogène, un groupe nitro, cyano, hydroxy, amino, alkylamino,en C1-C4, di-(alkyle en C1-C4)-amino, alkyle en C1-C6, halogénoalkyle en C1-C5, alcoxy en C1-C5, halogénoalcoxy en C1-C5, alkylthio en C1-C5, halogénoalkylthio en C1-C5, alcoxyalkyle en C2-C10, carboxyle, alcoxycarbonyle en C2-C6, alcanoyloxy en C2-C6, halogénoalcanoyloxy en C2-C6, (alkyle en C1-C4)-carbonylamino, (alkyle en C1-C4)-aminocarbonyle, di-(alkyle en C1-C4)aminocarbonyle, ou un groupe phényle éventuellement substitué par des halogènes, des groupes trifluorométhyle, nitro, cyano, amino, alcoxy en C1-C4, alkylthio en C1-C4 ou halogénoalkylthio en C1-C5, ou un groupe hétéroaryle tel que pyridyle, thiényle, furyle, benzymidazole-2-yle ou pyrimido-2-yle,
et en outre
$R^3$ et $R^4$ peuvent former ensemble et avec les deux atomes de carbone du noyau phényle auxquels ils sont reliés, un cycle benzénique ou naphtalénique éventuellement substitué par des halogénes, des groupes nitro, cyano, amino, hydroxy, trifluorométhyle, alkyle en C1-C5, alcoxy en C1-C5, halogénoalkylthio en C1-C5, halogénoalcoxy en C1-C5 ou alkylthio en C1-C5, ou bien un noyau hétérocyclique choisi parmi les noyaux 1,4-dioxanne, 1,3-dioxolanne, pyrazole, indole, thiophène, triazole et pipérazine, 2,2-diméthyl-1,3-dioxolanne, 1-méthylpyrazole, 1-méthyl-indole, N,N'-diméthylpipérazine, 2,2-diphényl-1,3-dioxolanne, 2-oxo-1,3-dioxolanne,
à l'exception de la 1-vinyl-benzoindazole-4,9-quinone.

**2.** Benzoindazole-4,9-quinones de formule I de la revendication 1, caractérisées en ce que $R^1$ représente un groupe alkyle en C2-C4 ou alcényle en C2-C4.

**3.** Benzoindazole-4,9-quinones de formule I de la revendication 1, caractérisées en ce que les symboles $R^2$ à $R^5$ représentent chacun, indépendamment les uns des autres, l'hydrogène, un groupe hydroxy ou un halogène.

**4.** Procédé de préparation des benzoindazole-4,9-quinones de formule I de la revendication 1, caractérisé en ce que l'on fait réagir des halogénures d'acides 5-aryl-méthylpyrazole-4-carboxyliques de formule

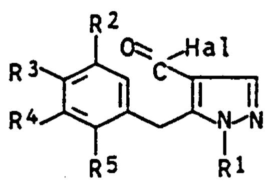

(II),

dans laquelle $R^1$, $R^2$, $R^3$, $R^4$ et $R^5$ ont les significations indiquées dans la revendication 1, et Hal représente un halogène, dans un agent solubilisant inerte, en présence d'un catalyseur de Friedel-Crafts, ce qui donne des composés de formule

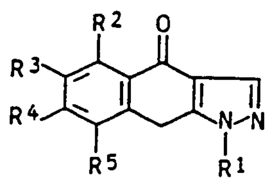

(III),

dans laquelle $R^1$, $R^2$, $R^3$, $R^4$ et $R^5$ ont les significations indiquées ci-dessus, qu'on convertit à l'aide d'agents aoxydants, de la manière habituelle et en présence d'un diluant, en les benzoindazole-4,9-quinones de formule I.

**5.** Produit herbicide contenant une benzoindazole-4,9-quinone de formule

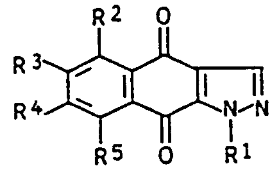

(Ia),

dans laquelle $R^1$, $R^2$, $R^3$, $R^4$ et $R^5$ ont les significations indiquées en référence à la formule I dans la revendication 1, $R^1$ pouvant en outre représenter l'hydrogène ou un groupe méthyle ou encore, lorsque $R^2$ à $R^5$ représentent l'hydrogène, un groupe vinyle.

**6.** Produit herbicide selon la revendication 5, caractérisé en ce qu'il contient des benzoindazole-4,9-quinones de formule Ia dans laquelle $R^1$ représente un groupe alkyle en C1-C4 ou alcényle en C2-C4.

**7.** Produit herbicide selon la revendication 5, caractérisé en ce qu'il contient des benzoindazole-4,9-quinones de formule Ia dans laquelle $R^2$ à $R^5$ représentent chacun, indépendamment les uns des autres, l'hydrogène, un groupe hydroxy, un halogène, un groupe nitro, cyano, amino, alkylamino en C1-C4 ou di-(alkyle en C1-C4)-amino.

**8.** Produit herbicide contenant des additifs inertes et une benzoindazole-4,9-quinonede formule Ia de la revendication 5.

**9.** Procédé pour combattre les croissances de végétaux indésirables, caractérisé en ce que l'on fait agir sur les végétaux et/ou leur habitat une benzoindazole-4,9-quinone de formule Ia de la revendication 5 en quantité herbicide efficace.

**Revendications pour l'Etat contractant suivant : ES**

**1.** Procédé de préparation des benzoindazole-4,9-quinones de formule

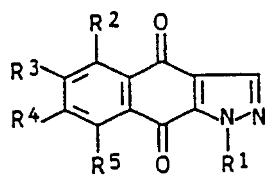

(I),

dans laquelle
$R^1$ représente un groupe alkyle en C2-C10, alcényle en C2-C10, alcynyle en C2-C10, halogénoalkyle en C1-C10, hydroxyalkyle en C1-C10, alcoxyalkyle en C2-C14, alkylthioalkyle en C2-C14, cycloalkyle en C3-C7, (alkyle en C1-C4)-carbonyloxy-alkyle en C1-C4, (alkyle en C1-C4)-aminocarbonyloxy-alkyle en C1-C4, phénylaminocarbonyloxy-alkyle en C1-C4 éventuellement substitué dans le groupe phényle par des halogènes des groupes alkyle en C1-C4, alcoxy en C1-C4, phénylsulfonyloxy-alkyle en C1-C4 éventuellement substitué dans la partie phényle par des halogènes, des groupes alkyle en C1-C4 ou alcoxy en C1-C4, (alcoxy en C1-C4)-carbonyl-alkyle en C1-C4, hydroxycarbonyl-alkyle en C1-C4, aminocarbonyl-alkyle en C1-C4, (alkyle en C1-C4)-aminocarbonyl-alkyle en C1-C4, di-(alkyle en C1-C4)-aminocarbonyl-alkyle en C1-C4, halogénoalcynyle en C3-C4, un groupe hétéroaryle éventuellement substitué par des halogènes ou des groupes alkyle en C1-C4 et contenant 1 ou 2 atomes d'azote, tel que pyrimidyle, pyridyle, imidazolyle, un groupe phényle éventuellement substitué par des halogénes, des groupes hydroxy, nitro, amino, alkylamino en C1-C4, di-(alkyle en C1-C4)-amino, cyano, alkyle en C1-C4, alcoxy en C1-C4, halogénoalcoxy en C1-C4, halogénoalkyle en C1-C4, alkylthio en C1-C4 ou halogénoalkylthio en C1-C4, ou un groupe benzyle éventuellement substitué par des halogènes, et
$R^2$, $R^3$, $R^4$ et $R^5$ représentent chacun, indépendamment les uns des autres, l'hydrogène, un halogène, un groupe nitro, cyano, hydroxy, amino, alkylamino,en C1-C4, di-(alkyle en C1-C4)-amino, alkyle en C1-C6, halogénoalkyle en C1-C5, alcoxy en C1-C5, halogénoalcoxy en C1-C5, alkylthio en C1-C5,

halogénoalkylthio en C1-C5, alcoxyalkyle en C2-C10, carboxyle, alcoxycarbonyle en C2-C6, alcanoyloxy en C2-C6, halogénoalcanoyloxy en C2-C6, (alkyle en C1-C4)-carbonylamino, (alkyle en C1-C4)-aminocarbonyle, di-(alkyle en C1-C4)-aminocarbonyle, ou un groupe phényle éventuellement substitué par des halogènes, des groupes trifluorométhyle, nitro, cyano, amino, alcoxy en C1-C4, alkylthio en C1-C4 ou halogénoalkylthio en C1-C5, ou un groupe hétéroaryle tel que pyridyle, thiényle, furyle, benzymidazole-2-yle ou pyrimido-2-yle,
et en outre,
$R^3$ et $R^4$ peuvent former ensemble et avec les deux atomes de carbone du noyau phényle auxquels ils sont reliés un noyau hétérocyclique choisi parmi les noyaux 1,4-dioxanne, 1,3-dioxolanne, pyrazole, indole, thiophène, triazole et pipérazine, 2,2-diméthyl-1,3-dioxolanne, 1-méthylpyrazole, 1-méthyl-indole, N,N'-diméthylpipérazine, 2,2-diphényl-1,3-dioxolanne, 2-oxo-1,3-dioxolanne ou un noyau benzénique ou naphtalénique éventuellement substitué par des halogènes, des groupes nitro, cyano, amino, hydroxy, trifluorométhyle, alkyle en C1-C5, alcoxy en C1-C5, halogénoalkylthio en C1-C5, halogénoalcoxy en C1-C5 ou alkylthio en C1-C5, à l'exception de la 1-vinyl-benzoindazole-4,9-quinone, caractérisé en ce que l'on fait réagir des halogénures d'acides 5-arylméthylpyrazole-4-carboxyliques de formule

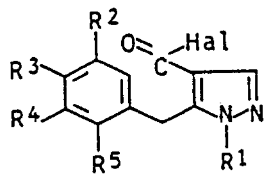

(II),

dans laquelle $R^1$, $R^2$, $R^3$, $R^4$ et $R^5$ ont les significations indiquées ci-dessus, et Hal représente un halogène, dans un agent solubilisant inerte, en présence d'un catalyseur de Friedel-Crafts, ce qui donne des composés de formule

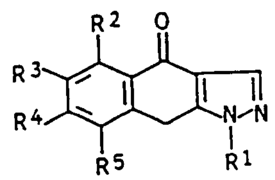

(III),

dans laquelle $R^1$, $R^2$, $R^3$, $R^4$ et $R^5$ ont les significations indiquées ci-dessus, qu'on convertit par des agents oxydants, de la manière habituelle, en présence d'un diluant en les benzoindazole-4,9-quinones de formule I.

**2.** Produit herbicide contenant une benzoindazole -4,9-quinone de formule

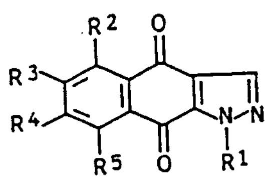

(Ia),

dans laquelle $R^1$, $R^2$, $R^3$, $R^4$ et $R^5$ ont les significations indiquées en référence à la formule I dans la revendication 1, $R^1$ pouvant en outre représenter l'hydrogène ou un groupe méthyle, ou bien encore, lorsque $R^2$ à R5 représentent l'hydrogène, un groupe vinyle.

**3.** Procédé pour combattre les croissances de végétaux indésirables, caractérisé en ce que l'on fait agir sur les végétaux et/ou leur habitat une benzoindazole-4,9-qui-none de formule la de la revendication 2 en quantité herbicide efficace.